# EUROPEAN PATENT APPLICATION

(11) **EP 2 371 381 A2**
(43) Date of publication of application: **05.10.2011**
(21) Application number: 11161768.4
(22) Date of filing: 10.07.2006
(51) Int. Cl.: A61K 39/12, A61K 39/00, A61P 31/12, A61P 35/00

(54) **Compositions and methods for eliciting an immune response to escape mutants of targeted therapies**

(30) Priority: 11.07.2005 US 698381 P
(62) Divisional of application: 06786760.6
(71) Applicant: Globeimmune, Inc., Louisville CO 80027 (US)
(72) Inventor: Rodell, Timothy C., Aspen, CO 81612 (US); Franzusoff, Alex, Nahant, MA 01908 (US); Apelian, David, Denver, CO 80209 (US)
(74) Representative: Roques, Sarah Elizabeth

(57) **Abstract**

Provided herein are cells, vectors and viruses in association with a mutant polypeptide that has emerged in response to a therapeutic or prophylactic agent; compositions comprising such cells, vectors and viruses and methods for their use in eliciting an immune response to the mutant polypeptide. In some examples, the immune response is a cellular immune response.

## Description

### CROSS-REFERENCES TO RELATED APPLICATIONS

The application is related to and claims benefit of U.S. Provisional Application Serial No.60/698,381, filed July 11,2005, the entire contents of which is hereby incorporated by reference herein in its entirety.

### GOVERNMENT RIGHTS

Not applicable.

### TECHNICAL FIELD

The present invention generally relates to compositions and methods for their use in eliciting an immune response to a mutant polypeptide, or to a cell that expresses the mutant polypeptide, wherein the mutant polypeptide is known to emerge or has emerged with a specific mutation in response to a targeted therapeutic or prophylactic drug agent(s). The compositions and methods disclosed herein are useful for eliciting an immune response, in particular, a cell mediated immune response, to the mutant polypeptide, including the deletion and/or arrest of a cell that expresses the mutant polypeptide, in conjunction with administration of the targeted drug agent. The compositions and methods may be used prophylactically or therapeutically.

### BACKGROUND OF THE INVENTION

Novel discoveries in cancer biology have provided the opportunity to design target-specific anti-cancer agents and fostered advancements in drug development. These discoveries make it possible to design molecules with high selectivity against specific targets in cancer cells. Segota & Bukowski, Cleveland Clinic J. Med., 2004, 71(7):551-560. For example, the success of the Bcr-Ab1 tyrosine kinase inhibitor imatinib (Gleevec) in the treatment of chronic myeloid leukemia (CML) has inspired great expectation for this targeted approach. Capdeville et al., Nature Reviews, 2002, 1:493-502. Targeted cancer therapy brings with it a critical problem: targets develop escape mutations ultimately leading to drug resistance. For example, it has been reported that mutations have been found to arise in patients that were initially responsive to treatment with Gleevec and who as a result of these mutations are refractory to further treatment with Gleevec. Gorre et al., Science 2001, 233:876-880; Shah et al., Cancer Cell 2002, 2:117-125; Branford et al., Blood 2002, 99(9):3742-3745; Deininger et al., Blood 2005, 105(7):2640-263. Similarly, mutations in epidermal growth factor receptor (EGFR) have been found in non-small cell lung cancer (NSCLC) patients that were reported to make them resistant to the action of therapeutic agents such as gefitinib (Iressa) or erlotinib (Tarceva) that specifically target EGFR. Kobayashi et al., N. Engl. J. Med., 2005, 352(8):786-792. Therefore, the effectiveness of these cancer drugs are significantly limited by the occurrence of escape mutations.

Similarly, although there are a variety of anti-viral compounds approved and being currently used for treatment of viral infections there is a continual, substantial problem with the development of drug resistance seen with the majority of these agents. For example, resistance to nucleoside reverse-transcriptase inhibitors used in the treatment of HIV has been reported. For example, mutations identified in HIV RT that are associated with resistance to stavudine include M41L, K65R, D67N, K70R, Q151M, L210W, T215Y/F, and K219E. Mutations identified in HIV RT that are associated with resistance to didanosine include K65R, L74V and M184V. Mutations identified in HIV RT that are associated with resistance to lamivudine include K65R and M184V/I. See for example, Johnson et al. (2005, International AIDS Society-USA vol. 13:51-57) and Schmitt et al. (2000, AIDS vol.14:653-658). Decreased susceptibility or complete resistance to specific antiviral drugs is a major factor limiting the efficacy of therapies against many retroviruses, RNA viruses and some DNA viruses, such as Hepatitis B virus, due to the error-prone nature of the viral reverse transcriptases or RNA-dependent RNA polymerases. It would be desirable to at least minimize the occurrence of mutants that emerge with the administration of therapeutic and/or prophylactic agents.

Methods for eliciting an immune response are disclosed in for example, Thyphronitis et al. (2004, Anticancer Research, vol. 24:2443-2454) and Plate et al. (2005) Journal of Cell Biology, vol. 94:1069). Yeast systems are disclosed in for example, U.S. Pat. No. 5,830,463, Stubbs et al.(2001, Nature Med. 5:625-629); Lu et al.(2004, Cancer Research 64:5084-5088); and Franzusoff, et al. (2005, Expert Opin. Bio. Ther. Vol.5:565-575).

All references cited herein, including patents, patent applications and publications, are hereby incorporated by reference in their entirety.

### BRIEF SUMMARY OF THE INVENTION

Provided herein are compositions comprising a yeast vehicle and a mutant polypeptide, a fragment thereof that comprises a mutation or a mimetope or nucleic acid that encodes for a mutant polypeptide, fragment thereof that comprises a mutation or a mimetope, wherein the mutant polypeptide is known to emerge or has emerged with at least one specific mutation in response to administration of a targeted therapeutic and/or prophylactic agent.

In some examples, the composition comprises one or more of the following:
i) a yeast vehicle comprising nucleic acid which encodes at least one mutant polypeptide, a fragment thereof that comprises a mutation, or a mimetope;
ii) a yeast vehicle comprising at least one mutant polypeptide, a fragment thereof that comprises a mutation, or a mimetope;
iii) a yeast vehicle in association with at least one mutant polypeptide, a fragment thereof that comprises a mutation, or a mimetope;
iv) a yeast vehicle comprising nucleic acid which encodes at least one mutant polypeptide, a fragment thereof that comprises a mutation, or a mimetope loaded intracellularly into a dendritic cell; or
v) a yeast vehicle and at least one mutant polypeptide, a fragment thereof that comprises a mutation, or mimetope loaded intracellularly into a dendritic cell,
wherein the mutant polypeptide is known to emerge or has emerged with at least one specific mutation in response to administration of a targeted therapeutic and/or prophylactic agent.

In some examples, the yeast vehicle is a whole yeast, a yeast spheroplast, a yeast cytoplast, a yeast ghost, or a subcellular yeast membrane extract or fraction thereof. In some examples the yeast vehicle is obtained from yeast selected from the group consisting of *Saccharomyces, Schizosaccharomyces, Kluveromyces, Hansenula, Candida* and *Pichia.* In some examples the yeast vehicle is obtained from *Saccharomyces,* such as S. *cerevisiae.*

In some examples the mutant polypeptide, fragment thereof that comprises a mutation, or mimetope comprises two or more epitopes. In other examples, the mutant polypeptide, fragment thereof that comprises a mutation, or mimetope comprises two or more epitopes comprising one or more mutations.

In some examples the mutant polypeptide, fragment thereof that comprises a mutation, or mimetope is encoded by a RNA or DNA virus. In some examples the virus is a retrovirus, flavivirus, reovirus, picornavirus, coronavirus, filovirus, rhabdovirus, bunyaviurs, orthomyxovirus, paramyxovirus, arenavirus, calicivirus, hepadnavirus, herpesvirus, poxvirus, adenovirus, parvovirus or papovavirus. In other examples the mutant polypeptide, fragment thereof that comprises a mutation, or mimetope is encoded by HIV, HBV or HCV.

Provided herein are compositions comprising a yeast vehicle and a mutant polypeptide, a fragment thereof that comprises a mutation or a mimetope or nucleic acid that encodes for a mutant polypeptide, fragment thereof that comprises a mutation or a mimetope, wherein the mutant polypeptide is known to emerge or has emerged with at least one specific mutation in response to administration of a targeted therapeutic and/or prophylactic agent.

In some examples the mutant polypeptide emerges in response to treatment with a targeted therapeutic and/or prophylactic agent selected from altazanavir, saquinavir, lamivudine, didnosine, embricitabine, zidovudine, stavudine, zalcitabine, abacavir, tenofovir, nevirapine, delavirdine, efavirenz, indinavir, ritonavir, nelfinavir, amprenavir, lopinavir, enfuvirtide, entecavir, adefovir, famciclovir, ganciclovir, interferon, ribavirin, thymosin-α, kinase inhibitors, nucleoside reverse transcriptase inhibitors, non-nucleoside reverse transcriptase inhibitors, viral protease inhibitors, viral entry/fusion inhibitors, viral protease inhibitors, nucleoside analogues or combinations thereof.

In some examples the mutant polypeptide, fragment thereof that comprises a mutation, or mimetope is encoded by an oncogene, is a tumor-associated antigen or is expressed by cancer cells. In some examples the mutant polypeptide, fragment thereof that comprises a mutation, or mimetope is MAGE, MAGE3, MAGMA6, MAGEA10, NY-ESO-1, gp100, tyrosinase, EGFR, FGFR, PSA, PSMA, VEGF, PDGFR, Kit, PMSA, CEA, Her2/neu, Muc-1, hTERT, Mart1, TRP-1, TRP-2, Bcr-Ab1, p53, p73, Ras, PTENSrc, p38, BRAF, adenomatous polyposis coli, myc, von Hippel landau protein, Rb-1, Rb-2, BRAC1, BRAC2, androgen receptor, Smad4, MDR1 or Flt3.

In some examples the mutant polypeptide emerges in response to treatment with a targeted therapeutic and/or prophylactic agent selected from imatinib, gefitinib, erlotinib, kinase inhibitors, SB 203580, tyrosine kinase inhibitors, or combinations thereof.

Provided herein are compositions comprising a yeast vehicle and a mutant polypeptide, a fragment thereof that comprises a mutation or a mimetope or nucleic acid that encodes for a mutant polypeptide, fragment thereof that comprises a mutation or a mimetope, wherein the mutant polypeptide is known to emerge or has emerged with at least one specific mutation in response to administration of a targeted therapeutic and/or prophylactic agent. In some examples the composition further comprises a pharmaceutically acceptable excipient. In some examples the composition further comprising at least one adjuvant. In other examples the adjuvant is selected from an agonist of Toll-like receptor, dinucleotide CpG sequences, single- or double-stranded RNA, Freund's adjuvant, a lipid moiety, mannans, glucans, aluminum-based salts, calcium-based salts, silica, polynucleotides, toxoids, serum proteins, viral coat proteins, gamma interferon, copolymers, Ribi adjuvants, or saponins and their derivatives.

Provided herein are methods for eliciting an immune response to a mutant polypeptide, a fragment thereof that comprises a mutation or a mimetope, or a cell that comprises nucleic acid encoding a mutant polypeptide and/or expresses the mutant polypeptide or a fragment thereof, wherein the mutant polypeptide is known to emerge or has emerged with a specific mutation in response to administration of a therapeutic and/or prophylactic drug agent(s) that comprise administering an effective amount of a composition in conjunction with the agent, wherein the composition comprises,
a. a cell, vector or virus comprising nucleic acid that encodes the mutant polypeptide;
b. a cell, vector or virus in association with the mutant polypeptide;
c. the mutant polypeptide, or a peptide (mimetope) that elicits an immune response to the mutant polypeptide; or
d. nucleic acid encoding the mutant polypeptide, or nucleic acid capable of binding to the nucleic acid, such as for example, siRNA or antisense RNA;
wherein an effective amount of the composition is administered in conjunction with the agent.

In some examples, the composition comprises one or more of the following:
i) a yeast vehicle comprising nucleic acid which encodes at least one mutant polypeptide, a fragment thereof that comprises a mutation, or a mimetope;
ii) a yeast vehicle comprising at least one mutant polypeptide, a fragment thereof that comprises a mutation, or a mimetope;
iii) a yeast vehicle in association with at least one mutant polypeptide, a fragment thereof that comprises a mutation, or a mimetope;
iv) a yeast vehicle comprising nucleic acid which encodes at least one mutant polypeptide, a fragment thereof that comprises a mutation, or a mimetope loaded intracellularly into a dendritic cell; or
v) a yeast vehicle and at least one mutant polypeptide, a fragment thereof that comprises a mutation, or a mimetope loaded intracellularly into a dendritic cell,
wherein the mutant polypeptide is known to emerge or has emerged with at least one specific mutation in response to administration of a targeted therapeutic and/or prophylactic agent.

In some examples, the immune response is a cellular immune response. In some examples, the immune response is an humoral response. In further examples, the immune response includes both cellular and humoral immune responses. In other examples, the composition further comprises an adjuvant, including but not limited to agonists for a Toll-like receptor (TLR); CpG nucleotide sequences; single- or double-stranded RNA; lipid moieties, such as lipopolysaccharide (LPS); mannans and glucans. In some examples, the composition comprises a yeast vehicle. In some examples, the yeast vehicle is a whole yeast, a yeast spheroplast, a yeast cytoplast, a yeast ghost, or a subcellular yeast membrane extract or fraction thereof. In some examples the yeast vehicle is obtained from yeast selected from the group consisting of *Saccharomyces, Schizosaccharomyces, Kluveroinyces, Hansenula, Candida* and *Pichia.* In some examples the yeast vehicle is obtained from *Saccharomyces,* such as *S. cerevisiae.*

In other examples, the mammal is a human. In further examples, the mutant polypeptide is expressed by a cancer cell or is a viral mutant polypeptide, such as for example, an HIV mutant polypeptide, a HCV mutant polypeptide or a HBV mutant polypeptide.

Provided herein are methods for ameliorating a symptom of a disease in a mammal, comprising administering to the mammal an effective amount of a composition in conjunction with a therapeutic and/or prophylactic agent, wherein a mutant polypeptide is known to emerge or has emerged with a specific mutation in response to administration of the agent, wherein the composition comprises,
a. a cell, vector or virus comprising nucleic acid that encodes the mutant polypeptide;
b. a cell, vector or virus in association with the mutant polypeptide;
c. the mutant polypeptide, or a peptide (mimetope) that elicits an immune response to the mutant polypeptide; or
d. nucleic acid encoding the mutant polypeptide, or nucleic acid capable of binding to the nucleic acid, such as for example, siRNA or antisense RNA;
wherein an effective amount of the composition is administered in conjunction with the agent. In some examples the disease is associated with the mutant polypeptide. In some examples, the immune response is a cellular immune response. In some examples the immune response is an humoral response. In other examples, the immune response includes both a cellular and humoral immune response.

Also provided herein are compositions comprising one or more of the following:
i) a yeast vehicle comprising nucleic acid which encodes at least one mutant polypeptide, a fragment thereof that comprises a mutation, or a mimetope;
ii) a yeast vehicle comprising at least one mutant polypeptide, a fragment thereof that comprises a mutation, or a mimetope;
iii) a yeast vehicle in association with at least one mutant polypeptide, a fragment thereof that comprises a mutation, or a mimetope;
iv) a yeast vehicle comprising nucleic acid which encodes at least one mutant polypeptide, a fragment thereof that comprises a mutation, or a mimetope loaded intracellularly into a dendritic cell; or
v) a yeast vehicle and at least one mutant polypeptide, a fragment thereof that comprises a mutation, or a mimetope loaded intracellularly into a dendritic cell,
wherein the mutant polypeptide is known to emerge or has emerged with at least one specific mutation in response to administration of a targeted therapeutic and/or prophylactic agent.

In other examples, the composition further comprises an adjuvant, including but not limited to agonists for a Toll-lilce receptor (TLR); CpG nucleotide sequences; single-or double-stranded RNA; lipid moieties, such as lipopolysaccharide (LPS); mannans and glucans. In some examples, the composition comprises a yeast vehicle. In some examples, the yeast vehicle is a whole yeast, a yeast spheroplast, a yeast cytoplast, a yeast ghost, or a subcellular yeast membrane extract or fraction thereof. In some examples the yeast vehicle is obtained from yeast selected from the group consisting of *Saccharomyces, Schizosaccharomyces, Kluveromyces, Hansenula, Candida* and *Pichia.* In some examples the yeast vehicle is obtained from *Saccharomyces,* such as *S. cerevisiae.* In some examples, the yeast vehicle is *Saccharomyces,* such as *S*. *cerevisiae.* In other examples, the mammal is a human. In further examples, the mutant polypeptide is expressed by a cancer cell or is a viral mutant polypeptide, such as for example, an HIV mutant polypeptide, a HCV mutant polypeptide or a HBV mutant polypeptide.

Provided herein are methods for reducing resistance to an agent administered to a mammal at risk of disease or infection or subject to disease or infection, whether the agent is administered prophylactically and/or therapeutically, comprising administering to the mammal an effective amount of a composition in conjunction with the agent, wherein said composition comprises,
a. a cell, vector or virus comprising nucleic acid that encodes the mutant polypeptide;
b. a cell, vector or virus in association with the mutant polypeptide;
c. the mutant polypeptide, or a peptide (mimetope) that elicits an immune response to the mutant polypeptide; or
d. nucleic acid encoding the mutant polypeptide, or nucleic acid, such as siRNA or anti-sense RNA that binds the nucleic acid,
wherein an effective amount of the composition is administered in conjunction with the agent. In some examples, the immune response is a cellular immune response. In further examples, the immune response is a cellular and humoral immune response.

In other examples, the composition further comprises an adjuvant, including but not limited to agonists for a Toll-like receptor (TLR); CpG nucleotide sequences; single-or double-stranded RNA; lipid moieties, such as lipopolysaccharide (LPS); mannans and glucans. In some examples, the composition comprises a yeast vehicle. In some examples, the yeast vehicle is a whole yeast, a yeast spheroplast, a yeast cytoplast, a yeast ghost, or a subcellular yeast membrane extract or fraction thereof. In some examples the yeast vehicle is obtained from yeast selected from the group consisting of *Saccharomyces, Schizosaccharomyces, Kluveromyces, Hansenula, Candida* and *Pichia*. In some examples the yeast vehicle is obtained from *Saccharomyces,* such as S *cerevisiae.* In some examples, the yeast vehicle is *Saccharomyces,* such as *S*. *cerevisiae.* In other examples, the mammal is a human. In further examples, the mutant polypeptide is expressed by a cancer cell or is a viral mutant polypeptide, such as for example, an HIV mutant polypeptide, a HCV mutant polypeptide or a HBV mutant polypeptide. In some examples of the methods, drug agents are disclosed herein in Table I, II and III. In other examples, a drug agent is an antibody or small molecule targeted to a cancer cell, a cell supporting cancer progression or a cell expressing a virus, or virus.

Provided herein are compositions capable of eliciting a cellular immune response comprising a vector or virus associated with a mutant polypeptide, wherein the mutant polypeptide is known to emerge or has emerged in response to administration of a therapeutic and/or prophylactic agent. In some examples, the composition comprises a yeast vehicle. In some examples, the yeast vehicle is a whole yeast, a yeast spheroplast, a yeast cytoplast, a yeast ghost, or a subcellular yeast membrane extract or fraction thereof. In some examples the yeast vehicle is obtained from yeast selected from the group consisting of *Saccharomyces, Schizosaccharomyces, Kluveromyces, Hansenula, Candida* and *Pichia.* In some examples the yeast vehicle is obtained from *Saccharomyces,* such as *S*. *cerevisiae.* In other examples, the composition comprises a yeast vehicle that comprises nucleic acid encoding a mutant polypeptide, a fragment thereof that comprises a mutation or a mimetope. In some examples, the composition comprises one or more of the following:
i) a yeast vehicle comprising nucleic acid which encodes at least one mutant polypeptide, a fragment thereof that comprises a mutation, or a mimetope;
ii) a yeast vehicle comprising at least one mutant polypeptide, a fragment thereof that comprises a mutation, or a mimetope;
iii) a yeast vehicle in association with at least one mutant polypeptide, a fragment thereof that comprises a mutation, or a mimetope;
iv) a yeast vehicle comprising nucleic acid which encodes at least one mutant polypeptide, a fragment thereof that comprises a mutation, or a mimetope loaded intracellularly into a dendritic cell; or
v) a yeast vehicle and at least one mutant polypeptide, a fragment thereof that comprises a mutation, or a mimetope loaded intracellularly into a dendritic cell,
wherein the mutant polypeptide is known to emerge or has emerged with at least one specific mutation in response to administration of a targeted therapeutic and/or prophylactic agent. In yet other examples, the mutant polypeptide is expressed by a cancer cell. In yet other examples, the mutant polypeptide is a viral mutant polypeptide. In further examples, the composition further comprises an adjuvant, including but not limited to agonists for a Toll-like receptor (TLR); CpG nucleotide sequences; single- or double-stranded RNA; lipid moieties, such as lipopolysaccharide (LPS); mannans and glucans. Provided herein are cells, such as dendritic cells that comprise a composition as described herein.

Provided herein are methods for preparing a yeast vehicle comprising a nucleic acid encoding for a mutant polypeptide, a fragment thereof that comprises a mutation, or a mimetope, wherein the mutant polypeptide is known to emerge or has emerged with at least one specific mutation in response to administration of a targeted therapeutic and/or prophylactic agent and wherein the nucleic acid is transfected into the yeast vehicle by a technique selected from the group consisting of diffusion, active transport, sonication, electroporation, microinjection, lipofection, adsorption and protoplast fusion. Provided herein are methods of preparing a yeast vehicle comprising a mutant polypeptide, a fragment thereof that comprises a mutation or a mimetope, wherein the mutant polypeptide is known to emerge or has emerged with at least one specific mutation in response to administration of a targeted therapeutic and/or prophylactic agent and wherein the mutant polypeptide, fragment thereof that comprises a mutation, or mimetope is placed into or associated with the yeast vehicle by a technique selected from the group consisting of diffusion, active transport, liposome fusion, sonication, electroporation, phagocytosis, lipofection, freeze/thaw cycling, chemical cross-linking, biological linking or mixing. In some methods the yeast vehicle is intracellularly loaded into a dendritic cell or a macrophage. In other methdos the yeast vehicle is intracellularly loaded into a dendritic cell.

Provided herein are uses of a composition in the preparation of a medicament. In addition provided are uses of a composition in the preparation of a medicament for use in eliciting an immune response to a mutant polypeptide, a fragment thereof that comprises a mutation, or a mimetope in a mammal. In some examples, the medicament is for use in eliciting an immune response to a mutant polypeptide, a fragment thereof that comprises a mutation, or a mimetope in a mammal in conjunction with a targeted therapeutic and/or prophylactic agent. In other examples, the medicament is for use in the treatment of a disease, wherein the disease is associated with a mutant polypeptide that has emerged in response to administration of a targeted therapeutic and/or prophylactic agent. In yet other examples, the medicament is for use in ameliorating a symptom of a disease, wherein the disease is associated with a mutant polypeptide that has emerged in response to administration of a targeted therapeutic and/or prophylactic agent. In some examples, the medicament is for use in reducing resistance to an agent administered to a mammal at risk of disease or infection or subject to disease or infection, wherein the disease or infection is associated with a mutant polypeptide that has emerged in response to administration of a targeted therapeutic and/or prophylactic agent.

Also provided herein are kits comprising a composition as described herein. In some examples a kit for eliciting an immune response to a mutant polypeptide, a fragment thereof that comprises a mutation, or a mimetope in a mammal comprises a yeast vehicle and at least one mutant polypeptide, fragments thereof that comprises a mutation, or mimetopes. In other examples the kit comprises a composition comprises one or more of the following:
i) a yeast vehicle comprising nucleic acid which encodes at least one mutant polypeptide, a fragment thereof that comprises a mutation, or a mimetope;
ii) a yeast vehicle comprising at least one mutant polypeptide, a fragment thereof that comprises a mutation, or a mimetope;
iii) a yeast vehicle in association with at least one mutant polypeptide, a fragment thereof that comprises a mutation, or a mimetope;
iv) a yeast vehicle comprising nucleic acid which encodes at least one mutant polypeptide, a fragment thereof that comprises a mutation, or a mimetope loaded intracellularly into a dendritic cell; or
v) a yeast vehicle and at least one mutant polypeptide, a fragment thereof that comprises a mutation, or a mimetope loaded intracellularly into a dendritic cell,
wherein the mutant polypeptide is known to emerge or has emerged with at least one specific mutation in response to administration of a targeted therapeutic and/or prophylactic agent. In some examples the kit further comprises a targeted therapeutic and/or prophylactic agent and/or instructional material for the use of the kit.

### BRIEF DESCRIPTION OF THE DRAWINGS

Not applicable.

### DETAILED DESCRIPTION OF THE INVENTION

There is a need for prophylactic and therapeutic drug agents that specifically target pathways that are unique to tumor cells and viruses, but there is a shortcoming in that the tumor cells and viruses undergo mutation(s) that allow them to escape the drug agent(s) and/or become resistant. Provided herein are compositions and methods for their use in eliciting an immune response to a mutant polypeptide that is known to emerge or that has emerged in response to a targeted drug agent, or to a cell that expresses the mutant polypeptide. In some examples, the immune response is a cellular immune response, in some examples the immune response is a humoral response, and in other example the immune response is both cellular and humoral. In some examples, the cellular immune response is intended to eliminate the cell, such as for example, a cancer cell, a cell that supports tumor progression or a cell that expresses a virus, that has escaped control and comprises mutations in the polypeptide targeted by the drug agent, but cell elimination is not required. In some examples, the cellular and/or humoral immune response will block cell proliferation or viral replication.

Accordingly, provided herein are methods for eliciting an immune response to a mutant polypeptide, or a cell that comprises nucleic acid encoding a mutant polypeptide and/or expresses a mutant polypeptide, that is known to emerge or has emerged with a specific mutation in response to administration of a therapeutic and/or prophylactic agent(s). In some examples, the immune response is a cellular immune response. In some examples the immune response is an humoral immune response. In other examples the immune response includes both a cellular and humoral immune response. In some examples, a mutant polypeptide is encoded by an oncogene and/or is expressed by a cancer cell. In some examples, a mutant polypeptide is associated with or expressed by a cancer cell. In some examples, the agent is targeted to the cancer cell. In some examples, the agent is a small molecule or antibody. In other examples, a mutant polypeptide is encoded by a virus and/or is expressed by a cell that is infected with a virus. In some examples, the mutant polypeptide is encoded by a pathogenic virus, such as for example, HIV, HCV or HBV. In some examples, the agent is targeted to the virus or cell expressing the virus.

Provided herein are methods for eliciting an immune response to a mutant polypeptide, or a cell that comprises nucleic acid encoding a mutant polypeptide and/or expresses the mutant polypeptide, wherein the mutant polypeptide is known to emerge or has emerged with a specific mutation in response to administration of a therapeutic and/or prophylactic agent(s) that comprise administering an effective amount of a composition in conjunction with the agent, wherein the composition comprises,
a. a cell, vector or virus comprising nucleic acid that encodes the mutant polypeptide;
b. a cell, vector or virus in association with the mutant polypeptide;
c. the mutant polypeptide, or a peptide (mimetope) that elicits an immune response to the mutant polypeptide; or
d. nucleic acid encoding the mutant polypeptide, or nucleic acid capable of binding to the nucleic acid, such as for example, siRNA or antisense RNA;
wherein an effective amount of the composition is administered in conjunction with the agent.

In some examples, the composition comprises one or more of the following:
i) a yeast vehicle comprising nucleic acid which encodes at least one mutant polypeptide, a fragment thereof that comprises a mutation, or a mimetope;
ii) a yeast vehicle comprising at least one mutant polypeptide, a fragment thereof that comprises a mutation, or a mimetope;
iii) a yeast vehicle in association with at least one mutant polypeptide, a fragment thereof that comprises a mutation, or a mimetope;
iv) a yeast vehicle comprising nucleic acid which encodes at least one mutant polypeptide, a fragment thereof that comprises a mutation, or a mimetope loaded intracellularly into a dendritic cell; or
v) a yeast vehicle and at least one mutant polypeptide, a fragment thereof that comprises a mutation, or a mimetope loaded intracellularly into a dendritic cell,
wherein the mutant polypeptide is known to emerge or has emerged with at least one specific mutation in response to administration of a targeted therapeutic and/or prophylactic agent.

In some examples, the composition is capable of eliciting a cellular immune response. In some examples, the composition is capable of eliciting an humoral response. In other examples, the cellular immune response further comprises a humoral immune response.

In some examples of the methods, the composition comprises an adjuvant. In yet other examples, the composition further comprises an agonist or ligand for a Toll-like receptor. In other examples, the composition comprises a yeast vehicle. In yet other examples, the composition comprises a CpG sequence. In other examples, the cell is a dendritic cell.

Provided herein are methods for ameliorating a symptom of a disease in a mammal, comprising administering to the mammal an effective amount of a composition in conjunction with a therapeutic and/or prophylactic agent, wherein a mutant polypeptide is known to emerge or has emerged with a specific mutation in response to administration of the agent, wherein the composition comprises,
a. a cell, vector or virus comprising nucleic acid that encodes the mutant polypeptide;
b. a cell, vector or virus in association with the mutant polypeptide;
c. the mutant polypeptide, or a peptide (mimetope) that elicits an immune response to the mutant polypeptide; or
d. nucleic acid encoding the mutant polypeptide, or nucleic acid capable of binding to the nucleic acid, such as for example, siRNA or antisense RNA;
wherein an effective amount of the composition is administered in conjunction with the agent.

In some examples, the composition comprises one or more of the following:
i) a yeast vehicle comprising nucleic acid which encodes at least one mutant polypeptide, a fragment thereof that comprises a mutation, or a mimetope;
ii) a yeast vehicle comprising at least one mutant polypeptide, a fragment thereof that comprises a mutation, or a mimetope;
iii) a yeast vehicle in association with at least one mutant polypeptide, a fragment thereof that comprises a mutation, or a mimetope;
iv) a yeast vehicle comprising nucleic acid which encodes at least one mutant polypeptide, a fragment thereof that comprises a mutation, or a mimetope loaded intracellularly into a dendritic cell; or
v) a yeast vehicle and at least one mutant polypeptide, a fragment thereof that comprises a mutation, or a mimetope loaded intracellularly into a dendritic cell,
wherein the mutant polypeptide is known to emerge or has emerged with at least one specific mutation in response to administration of a targeted therapeutic and/or prophylactic agent.

In some examples, the composition is capable of eliciting a cellular immune response. In some examples, the composition elicits an humoral immune response. In other examples, the cellular immune response further comprises a humoral immune response.

In some examples of the methods, the composition comprises an adjuvant. In yet other examples, the composition further comprises an agonist or ligand for a Toll-like receptor. In other examples, the composition comprises a yeast vehicle. In yet other examples, the composition comprises a CpG sequence. In other examples, the cell is a dendritic cell.

In some examples, the mammal is at risk for the disease and is administered the agent prophylactically; in other examples, the mammal is subject to the disease and the agent is administered therapeutically. In some examples the disease is cancer and in other examples the disease is an infectious disease caused by a pathogenic virus, such as for example HIV, HCV and HBV.

Also provided herein are cells, vectors, viruses, compositions, such as immunogenic compositions, and kits that comprise a mutant polypeptide, or nucleic acid encoding the mutant polypeptide, that is known to emerge or has emerged with a specific mutation in response to a therapeutic and/or prophylactic agent(s). In some examples, the composition is capable of eliciting an immune response when administered to a mammal. In some examples, the composition is capable of eliciting a cellular immune response when administered to a mammal. In some examples, the composition is capable of eliciting an humoral immune response when administered to a mammal. In other examples, the composition is capable of eliciting a cellular and humoral immune response when administered to a mammal.

Provided herein are yeast vehicles, including yeast vectors; yeast-based compositions; and methods for their use in eliciting an immune response to a mutant polypeptide, or a cell that comprises nucleic acid encoding the mutant polypeptide and/or that expresses the mutant polypeptide, that is known to emerge or has emerged with a specific mutation in response to a therapeutic and/or prophylactic agent(s). In some examples, the yeast vehicle comprises nucleic acid encoding a mutant polypeptide(s). In some examples, the yeast vehicle comprises nucleic acid expressing a mutant polypeptide(s). In other examples, the yeast vehicle is in association with the mutant polypeptide. In some examples, the yeast vehicle is loaded into a carrier cell, for example a dendritic cell or a macrophage. Also provided herein are methods for making such yeast vehicles and yeast-based compositions comprising them.

### General Techniques

The practice of the present invention will employ, unless otherwise indicated, conventional techniques of molecular biology (including recombinant techniques), immunology, cell biology, and biochemistry which are within the skill of the art. Such techniques are explained fully in the literature, such as, Molecular Cloning: A Laboratory Manual, third edition (Sambrook et al., 2001); Methods in Enzymology (Academic Press, Inc.); Current Protocols in Molecular Biology (F.M. Ausubel et al., eds., 1987 and annual updates); Fundamental Virology, second edition (ed. Fields et al. Raven press) and Current Protocols in Immunology (John Wiley & Sons, Inc., NY).

As used herein, the singular forms "a", "an" and "the" include plural references unless explicitly stated otherwise.

As used herein, "cancer" includes but is not limited to melanomas, squamous cell carcinoma, breast cancers, head and neck carcinomas, thyroid carcinomas, soft tissue sarcomas, bone sarcomas, testicular cancers, prostatic cancers, ovarian cancers, bladder cancers, skin cancers, brain cancers, angiosarcomas, hemangiosarcomas, mast cell tumors, primary hepatic cancers, lung cancers, pancreatic cancers, gastrointestinal cancers, renal cell carcinomas, hematopoietic neoplasias, and metastatic cancers, thereof.

As used herein, "infectious disease" refers to disease caused by infectious agents such as RNA viruses including but not limited to Retroviruses (such as for example, HIV), Flaviviruses (such as for example, HCV), Reoviruses, Picomaviruses, Coronaviruses, Filoviruses, Rhabdoviruses, Bunyaviruses, Orthomyxoviruses, Paramyxoviruses, Arenaviruses, Caliciviruses; and DNA viruses belonging to the families of Hepadnaviruses (such as for example, HBV), Herpesviruses, Poxviruses, Adenoviruses, Parvoviruses, and Papovaviruses.

As used herein, a therapeutic and/or prophylactic "agent" or "drug agent" that is targeted to a cell or virus means that the agent is directed to a molecule(s) associated with or functional for causing the transformation of a cell or supporting tumor progression, in the case of cancer, or associated with or functional for promoting or sustaining viral infection or viral replication. Examples of therapeutic and/or prophylactic agents that are designed to be targeted to a cell, such as a cancer cell or cell expressing a virus, or virus are disclosed herein and are known in the art.

As used herein, a "mutant polypeptide" encompasses a full length polypeptide encoded within a genome as well as a fragment thereof as long as the fragment comprises a mutation that is known to emerge or has emerged with a specific mutation in response to an agent, such as a prophylactic and/or therapeutic agent. Such mutant polypeptides that emerge with a specific mutation in response to an agent, such as a prophylactic and/or therapeutic agent targeted to a cell, such as a cancer cell or cell expressing virus, or virus, are generally referred to in the art as "escape mutants". A mutation can be found in any region of a full length polypeptide and may include an amino acid substitution, insertion or deletion or combination thereof, or fusion of non-sequential sequences, such as found in translocation events. In some examples, a mutant polypeptide that is known to emerge or that has emerged with a specific mutation in response to an agent is immunogenic on its own, that is, without being associated with an adjuvant, or other vector or vehicle, such as a yeast vehicle, but this is not required. In other examples, a mutant polypeptide that is known to emerge or that has emerged in response to an agent is immunogenic in association with an adjuvant that promotes its antigenicity.

As used herein, "adjuvants," include, for example, an agonist ligand for a Toll-like receptor (TLR) which elicits cytokine responses of innate immunity, and which are reported to be associated with maturation and activation of antigen presenting cells (APC); CpG nucleotide sequences; single- or double-stranded RNA (TLR7 agonists); lipid moieties, such as lipopolysaccharide (LPS); mannans and glucans, constituents of yeast (which are reported to function through interaction with TLRs 2, 4 and 6); and yeast vehicles, such as those described herein.

Administration of a mutant polypeptide or nucleic acid encoding the mutant polypeptide (which may be produced by any of the methods disclosed herein or known in the art) "in conjunction" with the agent is not intended to mean that the mutant polypeptide and agent are being administered simultaneously, although this is encompassed within the methods disclosed herein. A mutant polypeptide may be administered prior to, concurrently with or after administration of the agent, or a combination of the above. A mutant polypeptide or nucleic acid encoding the mutant polypeptide may be administered hours, days or months after the agent. In some examples, administration of a mutant polypeptide or nucleic acid encoding the mutant polypeptide (which may be produced by any of the methods disclosed herein or known in the art) is prior to administration of the agent, and may additionally be administered after administration of the agent, in particular if the agent is known to interfere directly or indirectly with proliferation of any cell type.

As used herein, a "mimetope" refers to a polypeptide that is able to mimic the ability of a mutant polypeptide to elicit an immune response, and in some examples, a cellular and /or humoral immune response, to the mutant polypeptide or cell expressing the mutant polypeptide. As used herein a "mimetope" includes, but is not limited to, a peptide that mimics one or more epitopes of a mutant polypeptide protein that is known to emerge or has emerged with a specific mutation in response to administration of a therapeutic and/or prophylactic agent; non-proteinaceous immunogenic portions of a mutant polypeptide (e.g., carbohydrate structures); and synthetic or natural organic molecules, including nucleic acids, that have a structure similar to at least one epitope of mutant polypeptide. Such mimetope can be designed using computer-generated structures of mutant polypeptides. Mimetopes can also be obtained by generating random samples of molecules, such as oligonucleotides, peptides or other organic molecules, and screening such samples for their ability to elicit an immune response, such as for example, a cellular immune response, by methods and assays as described herein and known in the art.

As used herein, "ameliorating a symptom of a disease or infection" includes palliating, stabilizing, reversing, slowing or delaying any symptom and/or progression of the disease state, which may be measured by clinical and/or sub-clinical criteria.

In some examples, an "effective amount" of a mutant polypeptide (or mimetope thereof), or nucleic acid encoding the mutant polypeptide refers to an amount capable of eliciting an immune response when administered to a mammal. In some examples, the immune response is a cellular immune response. In other examples the immune response is a humoral response. In some examples the immune response is a cellular and humoral response.

As used herein, "mammal", "mammalian" or "mammalian host" includes human and non-human primates such as chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The term does not denote a particular age. Thus, adult, juvenile, and newborn individuals are intended to be covered, as well as pre-natal mammals.

An "antigen" refers to a molecule containing one or more epitopes (either linear, conformational or both) or immunogenic determinants that will stimulate a host's immune-system, such as a mammal's immune system, to make an antigen-specific humoral and/or cellular antigen-specific response. An antigen may be an "immunogen" by itself or in conjunction with an agent that promotes its antigenicity. The term "antigen" includes whole protein, truncated protein, fragment of a protein and peptide. Antigens may be naturally occurring, or genetically engineered variants of the protein. The term "antigen" includes subunit antigens, (i.e., antigens which are separate and discrete from a whole organism with which the antigen is associated in nature). Antibodies such as anti-idiotype antibodies, or fragments thereof, and synthetic peptide mimetopes, that is synthetic peptides which can mimic an antigen or antigenic determinant, are also captured under the definition of antigen as used herein. In some examples, antigen encompasses a mutant polypeptide or is obtainable from a mutant polypeptide that is known to emerge or has emerged with a specific mutation in response to a prophylactic and/or therapeutic agent, and may be naturally occurring or synthetic. An antigen can be as small as a single epitope, or larger, and can include multiple epitopes. As such, the size of an antigen can be as small as about 5-12 amino acids (e. g., a peptide) and as large as a full length protein, including multimers and fusion proteins, chimeric proteins, whole cells, whole microorganisms, or portions thereof (e. g., lysates of whole cells or extracts of microorganisms). It will be appreciated that in some examples (i. e., when the antigen is expressed by a vector, such as a yeast vector, or virus from a recombinant nucleic acid molecule), the antigen includes, but is not limited to a protein, or fragment thereof, fusion protein, chimeric protein, multimers, rather than an entire cell or microorganism.

As used herein, "epitope" is defined herein as a single antigenic site within a given antigen that is sufficient to elicit an immune response, which may be a cellular and/or humoral immune response. Those of skill in the art will recognize that T-cell epitopes are different in size and composition from B-cell epitopes, and that epitopes presented through the Class I major histocompatibility complex (MHC) pathway differ from epitopes presented through the Class II MHC pathway. Generally, a B-cell epitope will include at least about 5 amino acids but can be as small as 3-4 amino acids. A T-cell epitope, such as a cytotoxic T-lymphocyte (CTL) epitope, will include at least about 7-9 amino acids, and a helper T-cell epitope at least about 12-20 amino acids. Normally, an epitope will include between about 7 and 15 amino acids, such as, 8, 9, 10, 12 or 15 amino acids.

As used herein, an "immunological response or "immune response" to a mutant polypeptide (or mimetope thereof) or nucleic acid encoding the polypeptide (or nucleic acid capable of binding to the mutant polypeptide, such as for example, siRNA or antisense RNA), or composition comprising a polypeptide or nucleic acid, includes the development in a mammal of a cellular immune response that recognizes the polypeptide. In some examples, the immune response is an humoral immune response. In some examples, the cellular immune response additionally includes an humoral immune response. The immune response may be specific to the mutant polypeptide, but this is not required. The immune response that is elicited by administration of a mutant polypeptide, or nucleic acid encoding the polypeptide, can be any detectable increase in any facet of the immune response (e. g., cellular response, humoral response, cytokine production), as compared to the immune response in the absence of the administration of the polypeptide or nucleic acid. An immune response may be a mutant polypeptide specific response, but this is not required. Encompassed within the present invention are compositions in association with a mutant polypeptide (or mimetope thereof), or nucleic acid encoding the mutant polypeptide that elicit the immune response.

As used herein, an "humoral immune response" refers to an immune response mediated by antibody molecules or immunoglobulins. Antibody molecules of the present invention include the classes of IgG (as well as subtypes IgG1, IgG2a, and IgG2b), IgM, IgA, IgD, and IgE. Antibodies functionally include antibodies of primary immune response as well as memory antibody responses or serum neutralizing antibodies. With respect to infectious disease, antibodies of the present invention may serve to, but are not required to, neutralize or reduce infectivity of the virus encoding the mutant polypeptide, and/or mediate antibody-complement, or antibody dependent cell cytotoxicity (ADCC) to the mutant polypeptide.

As used herein, a "cellular immune response" is one mediated by T-lymphocytes and/or other white blood cells, including without limitation natural killer (NK) cells and macrophage. T-lymphocytes of the present invention include T-cells expressing alpha/beta T-cell receptor subunits or gamma/delta receptor expressing T-cells and may be either effector or suppressor T-cells.

As used herein, "T-lymphocytes" or "T-cells" are non-antibody producing lymphocytes that constitute a part of the cell-mediated arm of the immune system. T-cells arise from immature lymphocytes that migrate from the bone marrow to the thymus, where they undergo a maturation process under the direction of thymic hormones. Maturing T cells become immunocompetent based on their ability to recognize and bind a specific antigen. Activation of immunocompetent T cells is triggered when an antigen binds to the lymphocyte's surface receptors. It is known that in order to generate T cell responses, antigen must be synthesized within or introduced into cells, subsequently processed into small peptides by the proteasome complex, and translocated into the endoplasmic reticulum/Golgi complex secretory pathway for eventual association with major histocompatibility complex (MHC) class I proteins. Alternatively, peptide antigens may be introduced from the outside of cells to displace peptides already bound into MHC-I or MHC-II receptors. Functionally cellular immunity includes antigen-specific cytotoxic T-lymphocytes cells (CTL).

As used herein, "antigen-specific killer T cells", "CTL", or "cytotoxic T-cells" as used herein refer to cells which have specificity for peptide antigens presented in association with proteins of the MHC or human leukocyte antigens (HLA) as the proteins are referred to in humans. CTLs of the present invention include activated CTL which have become triggered by specific antigen in the context of MHC; and memory CTL or recall CTL to refer to T cells that have become reactivated as a result of re-exposure to antigen as well as cross-reactive or cross clade CTL. CTLs of the present invention include CD4+ and CD8+ T cells. Activated antigen-specific CTLs of the present invention promote the destruction and/or lysis of cells of the subject infected with the pathogen to which the CTL are specific, blocking pathogen entry via secretion of chemokines and cytokines including without limitation macrophage inflammatory protein 1 a (MTP-1a), MIP-1B, and RANTES; and secretion of soluble factors that suppress infections. Cellular immunity of the present invention also refers to antigen-specific response produced by the T helper subset of T cells. Helper T cells act to help stimulate the function, and focus the activity of nonspecific effector cells against cells displaying peptide in association with MHC molecules on their surface. A cellular immune response also refers to the production of cytokines, chemokines and other such molecules produced by activated T-cells and/ or other white blood cells including those derived from CD4 and CD8 T cells and NK cells. A composition that elicits a cellular immune response may serve to sensitize a mammal by the presentation of the polypeptide in association with MHC molecules at the cell surface. The cell-mediated immune response is directed at, or near, cells presenting antigen at their surface. In addition, antigen-specific T-lymphocytes can be generated to allow for the future protection of an immunized host.

The ability of a particular polypeptide or antigen to stimulate a cell-mediated immunological response may be determined by a number of assays known in the art, such as by lymphoproliferation (lymphocyte activation) assays, CTL killing assays, or by assaying for T-lymphocytes specific for the antigen in a sensitized subject. Such assays are well known in the art. See, e.g., Erickson et al., J. Immunol. (1993) 151:4189-4199; Doe et al., Eur. J. Immunol. (1994) 24:2369-2376. Other methods of measuring cell-mediated immune responses include measurement of intracellular cytokine or cytokines secretion by T-cell populations, or by measurement of epitope-specific T-cells (e.g., by the tetramer technique) (reviewed by McMichael, A. J., and O'Callaghan, C. A., J. Exp. Med. 187(9)1367-1371, 1998; Mcheyzer-Williams, M. G., et al, Immunol, Rev. 150:5-21, 1996; Lalvani, A., et al, J. Exp. Med. 186:859-865, 1997).

As used herein, an "immunological response", or "immune response" encompasses one which stimulates the production of CTLs, and/or the production or activation of helper T-cells and/or an antibody "mediated immune response. T lymphocytes" or "T cells" are non-antibody producing lymphocytes that constitute a part of the cell-mediated arm of the immune system. T cells arise from immature lymphocytes that migrate from the bone marrow to the thymus, where they undergo a maturation process under the direction ofthymic hormones. Here, the mature lymphocytes rapidly divide increasing to very large numbers. The maturing T cells become immunocompetent based on their ability to recognize and bind a specific antigen. Activation of immunocompetent T cells is triggered when an antigen binds to the lymphocytes surface receptors in the context of presentation by MHC/HLA receptors and co receptors.

As used herein, an "immunogenic composition" is a composition that comprises a mutant polypeptide (or mimetope thereof) or nucleic acid encoding the mutant polypeptide that is known to emerge or has emerged with a specific mutation in response to a therapeutic and/or prophylactic agent, and may or may not comprise an adjuvant that promotes the antigenicity of a mutant polypeptide, wherein administration of the composition to a mammal results in the development of a cellular immune response, an humoral immune response or a cellular and humoral immune response, An immunogenic composition includes a composition that is capable of eliciting a protective cellular immune response, but this is not required.

As used herein, "prophylactic composition" refers to a composition administered to a mammalian subject or host that is "immunologically naïve" or has not been previously exposed to the antigen of the pathogen or one that has not generated an effective immune response to the pathogen to prevent disease, such as cancer and infection or re-infection (however the present invention does not require that the infection or re-infection is completely prevented). Prophylactic compositions of the present invention do not necessarily generate sterilizing immunity in the host or subject to which they have been administered.

As used herein, "therapeutic composition" refers to a composition administered to a subject or host that is subject to cancer or is or has been infected with a virus, in the case of infectious disease, and in some examples, has progressed to a disease state.

As used herein, the term "immunization" "immunize" or "immunized", refers to the process of administering an immunogenic composition to a live mammalian subject or host in an amount effective to induce an immune response to the composition. In some examples, the immune response includes a cellular immune response, e.g., a cytotoxic T cell response. In some examples, the immune response includes an humoral response, e.g. antibody production. In some examples, the immune response includes both a cellular and humoral response.

### Vectors and Compositions

Provided herein are vectors, such as for example, bacterial, viral, insect and yeast vectors comprising nucleic acid encoding a mutant polypeptide that is known to emerge or has emerged with a specific mutation in response to an agent. Provided herein are compositions that comprise such vectors and viruses in association with the mutant polypeptide. Such vectors and viruses include but are not limited to bacterial vectors, naked plasmid DNA, yeast vectors and vehicles, rAAV, adenoviruses, canarypox viruses, pox viruses, vaccinia virus, modified vaccinia Ankara (MVA), alphaviruses, rhabdoviruses, Venezuela equine encephalitis virus, and baculovirus and can be produced by methods deemed routine to the skilled artisan. A number of viral based systems have been developed for gene transfer into mammalian cells. For example, the life cycle and genetics of AAV are reviewed in Muzyczka, Current Topics in Microbiology and Immunology, 158: 97-129 (1992). Additional disclosure on AAV systems are provided in, for example, RO Snyder, et al., Human Gene Therapy 8:1891-1900, 1997; D Duan, et al., J.Virol. 8568-8577, 1998; Duan D, et al., J Virol. 73:161-169, 1999; N Vincent-Lacaze, et al., J Virol. 73:1949-1955,1999; C McKeon, et al., NIDDK Workshop on AAV Vectors: Gene transfer into quiescent cells. Human Gene Therapy 7:1615-1619, 1996; HNakai, et al., J Virol 75:6969-6976, and 2001; BC Schnepp, et al., J. Viral. 2003 77: 3495-350. AAV systems are described in for example, U.S. Pat. No. 5,786,211; U.S. Pat. No. 5,871,982; and U.S. Pat. No. 6,258,595. A number of adenovirus vectors and expression systems have been described. See for example Haj-Ahmad and Graham, J. Virol. (1986) 57:267-274; Bett et al., J. Virol. (1993) 67:5911-5921; Mittereder et al., Human Gene Therapy (1994) 5:717-729; Seth et al., J. Virol. (1994) 68:933-940; Barr et al., Gene Therapy (1994) 1:51-58; Berkner, K. L. BioTechniques (1988) 6:616-629; and Rich et al., Human Gene Therapy (1993) 4:461-476). Poxvirus vaccines are described in for example, Small, Jr., P. A., et al. (U.S. Pat. No. 5,676,950, issued Oct. 14, 1997). Additional viral vectors include those derived from the pox family of viruses, including vaccinia virus and avian poxvirus. Baculovirus is described in for example, U.S. Pat. No. 5,731,182.

Provided herein are methods for eliciting an immune response, in some examples, a cellular immune response, to a mutant polypeptide, or nucleic acid encoding the polypeptide. In some examples, the immune response is an humoral response and in other examples the immune response includes both a cellular and humoral immune response. Illustrative examples of mutant polypeptides encoded by oncogenes and viruses that are known to emerge with specific mutations in response to therapeutic and/or prophylactic agents are disclosed herein and others are known in the art. Emergence of mutant polypeptides in response to such agent(s) is believed to be associated with, for example, decreased susceptibility and/or resistance to the agent(s) and/or an increase in a clinical and/or sub-clinical symptom of the disease, such as for example, in HIV, presence of a decreased CD4 count. The benefit of eliciting an immune response, such as for example a cellular immune response, to a mutant polypeptide that is known to emerge or has emerged with a specific mutation in response to an agent targeted to a cell or virus that will be, has been or is being administered to a mammal may include extending the duration of the agent effectiveness; minimizing or reversing resistance to the agent; delaying or minimizing emergence of the specific mutant polypeptide; eliminating the specific mutant polypeptide (although elimination is not required in order to have a benefit); and minimizing, reducing or reversing a symptom of the disease, and/or slowing progression of the disease.

Provided herein are vectors, including for example, but not limited to bacterial, viral, insect and yeast vectors, comprising nucleic acid that encodes a mutant polypeptide that is known to emerge or has emerged with a specific mutation in response to a therapeutic and/or prophylactic agent(s) as well as compositions comprising them. In some examples, the vector is a yeast vector. In other examples, vectors comprise nucleic acid capable of binding to the mutant polypeptide, such as for example, siRNA or antisense RNA, thereby inhibiting expression of the mutant polypeptide. Provided herein are compositions comprising vectors, including for example, bacterial, viral, insect and yeast vectors, in association with a mutant polypeptide, a fragment thereof that comprises a mutation, or mimetope thereof, that is known to emerge or has emerged with a specific mutation in response to a therapeutic and/or prophylactic agents). In some examples, the vector is a yeast vector or vehicle. In other examples, compositions that comprise a mutant polypeptide further comprise an adjuvant. Examples of adjuvants are described herein and are known in the art. Provided herein are cells, including for example, but not limited to, dendritic cells, that comprise nucleic acid encoding a mutant polypeptide as described herein and/or vectors or virus that express the mutant polypeptide. In some examples, the dendritic cells comprise a mutant polypeptide as described herein. Also provided herein are compositions comprising nucleic acid encoding the mutant polypeptide. Also provided herein are methods for making such cells, vectors, viruses and compositions.

A mutant polypeptide or fragment thereof that comprises a mutation, or nucleic acid encoding the polypeptide or fragment, and/or compositions or vectors comprising them, is administered in conjunction with a prophylactic and/or therapeutic agent and may be administered to a mammal that is at risk for cancer or infection, or a mammal subject to cancer or infection. A mutant polypeptide or fragment thereof that comprises a mutation, or nucleic acid encoding the polypeptide or fragment, and/or compositions or vectors comprising them can be administered after the agent, whether or not the mutant polypeptide has emerged in response to the agent, and whether or not resistance to the agent has been detected. Detection of emergence of a mutant polypeptide in response to an agent can be determined by methods known in the art. If a mutant polypeptide, or nucleic acid encoding it, and/or compositions or vectors comprising them, is administered to a mammal in conjunction with an agent and it has been determined that a mutant polypeptide has emerged with a specific mutation in response to an agent, the methods described herein do not require ablation of the mutant polypeptide in order for the mammal to benefit from administration.

Accordingly, a mutant polypeptide, a fragment thereof that comprises a mutation, or a mimetope, or nucleic acid encoding it and/or compositions or vectors comprising them can be administered in conjunction with an agent in methods as described herein including methods for eliciting an immune response, such as for example, a cellular immune response, an humoral immune response or both; methods for ameliorating the symptoms of cancer or infection in a mammal; methods for reducing resistance to an agent; methods for increasing the efficacy of an agent; methods for increasing susceptibility to the agent; methods for delaying or minimizing emergence of a mutant polypeptide; and methods for decreasing clinical and/or sub-clinical symptoms of cancer or infection. In some examples, administration of a mutant polypeptide, or nucleic acid encoding it, and/or compositions or vectors comprising them, in conjunction with an agent delays or minimizes emergence of a mutant polypeptide and/or increases the efficacy of the agent and/or decreases clinical and/or sub-clinical symptoms of cancer or infection to a greater extent as compared to administration of the agent alone, that is, without administration of the mutant polypeptide, or nucleic acid encoding it, and/or compositions or vectors comprising them.

### Yeast-based compositions and methods

Provided herein are yeast vectors, yeast vehicles and yeast-based compositions that comprise a mutant polypeptide that is known to emerge or has emerged with a specific mutation in response to an agent. As used herein, the term "yeast vector" and "yeast vehicle" are used interchangeably and include, but are not limited, to whole yeast, a yeast spheroplast, a yeast cytoplast, a yeast ghost, and a subcellular yeast membrane extract or fraction thereof. In some examples, a yeast cell or yeast spheroplast is used to prepare the yeast vehicle, which in some examples comprises nucleic acid molecule encoding the mutant polypeptide, such that the polypeptide is expressed by the yeast cell or yeast spheroplast. In some examples, the yeast vehicle is obtainable from a non-pathogenic yeast. In another examples, the yeast vehicle is obtainable from a yeast selected from the group consisting of: *Saccharomyces, Schizosaccharomyces, Kluveromyces, Hansenula, Candida* and *Pichia.* In some examples, the *Saccharomyces* is *S*. *cerevisiae.*

In general, the yeast vehicle and mutant polypeptide can be associated by any technique described herein. In some examples, the yeast vehicle is loaded intracellularly with a mutant polypeptide. In other examples, the mutant polypeptide is covalently or non-covalently attached to the yeast vehicle. In yet additional examples, the yeast vehicle and the mutant polypeptide are associated by mixing. In other examples, the mutant polypeptide is expressed recombinantly by the yeast vehicle or by the yeast cell or yeast spheroplast from which the yeast vehicle was derived.

Accordingly, provided herein are yeast vehicles which encompass any yeast cell (e.g., a whole or intact cell) or a derivative thereof that can be used in conjunction with a mutant polypeptide in a composition, or as an adjuvant. The yeast vehicle can therefore include, but is not limited to, a live intact yeast microorganism (i.e., a yeast cell having all its components including a cell wall), a killed (dead) intact yeast microorganism, or derivatives thereof including: a yeast spheroplast (i.e., a yeast cell lacking a cell wall), a yeast cytoplast (i.e., a yeast cell lacking a cell wall and nucleus), a yeast ghost (i.e., a yeast cell lacking a cell wall, nucleus and cytoplasm), or a subcellular yeast membrane extract or fraction thereof (also referred to previously as a subcellular yeast particle).

Yeast spheroplasts are typically produced by enzymatic digestion of the yeast cell wall. Such a method is described, for example, in Franzusoff et al., 1991, Meth. Enzymol. 194, 662-674., incorporated herein by reference in its entirety. Yeast cytoplasts are typically produced by enucleation of yeast cells. Such a method is described, for example, in Coon, 1978, Natl. Cancer Inst. Monogr. 48, 45-55 incorporated herein by reference in its entirety. Yeast ghosts are typically produced by resealing a permeabilized or lysed cell and can, but need not, contain at least some of the organelles of that cell. Such a method is described, for example, in Frauzusoff et al., 1983, J. Biol. Chem. 258, 3608-3614 and Bussey et al., 1979, Biochim. Biophys. Acta 553, 185-196, each of which is incorporated herein by reference in its entirety. A subcellular yeast membrane extract or fraction thereof refers to a yeast membrane that lacks a natural nucleus or cytoplasm. The particle can be of any size, including sizes ranging from the size of a natural yeast membrane to microparticle produced by sonication or other membrane disruption methods known to those skilled in the art, followed by resealing. A method for producing subcellular yeast membrane extracts is described, for example, in Franzusoff et al., 1991, Meth. Enzymol. 194, 662-674. One may also use fractions of yeast membrane extracts that contain yeast membrane portions and, when the antigen is expressed recombinantly by the yeast prior to preparation of the yeast membrane extract, the antigen of interest is part of the extract. Yeast can also be electroporated or otherwise loaded with target antigens, such as peptides.

Any yeast strain can be used to produce a yeast vehicle of the present invention. Yeast are unicellular microorganisms that belong to one of three classes: Ascomycetes, Basidiomycetes and Fungi Imperfecti. While pathogenic yeast strains, or nonpathogenic mutants thereof can be used, in some examples, nonpathogenic yeast strains are used. Genera of yeast strains for use in the compositions and methods disclosed herein include *Saccharomyces, Candida* (which can be pathogenic), *Cryptococcus, Hansenula, Kluyveromyces, Pichia, Rhodotorula, Schizosaccharomyces* and *Yarrowia.* In some examples, yeast strains include *Saccharomyces, Candida, Hansenula, Pichia* and *Schizosaccharomyces.* In some examples, a yeast strain is *Saccharomyces.* Species of yeast strains include *Saccharomyces cerevisiae, Saccharomyces carlsbergensis, Candida albicans, Candida kefyr, Candida tropicalis, Cryptococcus laurentii, Cryptococcus neoformans, Hansenula anomala, Hansenula polymorpha, Kluyveromyces fragilis, Kluyveromyces lactis, Kluyveromyces marxianus* var. *lactis, Pichia pastoris, Rhodotorula rubra, Schizosaccharomyces pombe,* and *Yarrowia lipolytica.* It is to be appreciated that a number of these species include a variety of subspecies, types, subtypes, etc. that are meant to be included within the aforementioned species. In some examples, yeast species include S. *cerevisiae, C. albicans, H. polymorpha, P. pastoris* and S. *pombe.* In some examples, S. *cerevisiae* is used due to its being relatively easy to manipulate and being "Generally Recognized As Safe" or "GRAS" for use as food additives (GRAS, FDA proposed Rule 62FR18938, April 17, 1997). In some examples, a yeast strain that is capable of replicating plasmids to a particularly high copy number, such as a *S*. *cerevisiae* cir° strain is used. Other useful strains are known in the art.

In some examples, a yeast vehicle of the present invention is capable of fusing with the cell type to which the yeast vehicle and mutant polypeptide is being delivered, such as a dendritic cell or macrophage, thereby effecting particularly efficient delivery of the yeast vehicle, and in many examples, the antigen, to the cell type. As used herein, fusion of a yeast vehicle with a targeted cell type refers to the ability of the yeast cell membrane, or particle thereof, to fuse with the membrane of the targeted cell type (e.g., dendritic cell or macrophage), leading to syncytia formation. As used herein, a syncytium is a multinucleate mass of protoplasm produced by the merging of cells. A number of viral surface proteins (including some HIV proteins) and other fusogens (such as those involved in fusions between eggs and sperm) have been shown to be able to effect fusion between two membranes (i.e., between viral and mammalian cell membranes or between mammalian cell membranes). For example, a yeast vehicle that produces an HIV gp120/gp41 heterologous antigen on its surface is capable of fusing with a CD4+ T-lymphocyte. It is noted, however, that incorporation of a targeting or fusogenic moiety into the yeast vehicle, while it may be desirable under some circumstances, is not required. It has been shown that yeast vehicles are readily taken up by dendritic cells (as well as other cells, such as macrophage).

Yeast vehicles can be formulated into yeast-based compositions, including compositions intended for direct administration to individuals subject to or at risk for cancer or infection directly or first *ex vivo* loaded into a carrier such as a dendritic cell, using a number of techniques known to those skilled in the art, prior to administration.

Provided herein are yeast vehicles, and compositions comprising them, that comprise at least one mutant polypeptide for administration to a mammal. In some examples, the composition comprises one or more of the following:
i) a yeast vehicle comprising nucleic acid which encodes at least one mutant polypeptide, a fragment thereof that comprises a mutation, or a mimetope,;
ii) a yeast vehicle comprising at least one mutant polypeptide, a fragment thereof that comprises a mutation, or a mimetope;
iii) a yeast vehicle in association with at least one mutant polypeptide, a fragment thereof that comprises a mutation, or a mimetope;
iv) a yeast vehicle comprising nucleic acid which encodes at least one mutant polypeptide, a fragment thereof that comprises a mutation, or a mimetope loaded intracellularly into a dendritic cell; or
v) a yeast vehicle and at least one mutant polypeptide, a fragment thereof that comprises a mutation, or a mimetope loaded intracellularly into a dendritic cell,
wherein the mutant polypeptide is known to emerge or has emerged with at least one specific mutation in response to administration of a targeted therapeutic and/or prophylactic agent.

Such compositions can include, one, two, a few, several or a plurality of mutant polypeptides (such as those described herein in Table II for HIV) including one or more immunogenic domains of one or more mutant polypeptides, as desired. As used herein, polypeptide includes "antigen". As used herein, an antigen, includes any portion of a protein (peptide, protein fragment, full-length protein), wherein the protein is naturally occurring or synthetically derived, a cellular composition (whole cell, cell lysate or disrupted cells), an organism (whole organism, lysate or disrupted cells), a carbohydrate, a lipid, or other molecule, or a portion thereof, wherein the antigen elicits an antigen-specific immune response (humoral and/or cellular immune response).

Yeast exhibit many of the particulate features of immunostimulatory complexes, with the added advantage that they naturally possess adjuvant-like properties and can be easily engineered to express multiple polypeptides, including antigens. Lu et al., (2004) Cancer Research 64, 5084-5088, demonstrated that a yeast-based immunotherapy was capable of eliciting cell-mediated immune responses to tumors expressing Ras oncoproteins harboring a single amino acid mutation. The results demonstrated the ability of yeast vehicles and yeast-based systems to target immunotherapy against polypeptides bearing single amino acid mutations. Accordingly, provided herein are yeast vehicles and yeast-based compositions comprising a mutant polypeptide(s) that is known to emerge or which has emerged in response to an agent, and methods for using them to elicit an immune response to the mutant polypeptide. In some examples, the immune response is a cellular immune response. In some examples, the immune response is an humoral response. In other examples, the immune response is both cellular and humoral. In some further examples, the yeast vehicle is engineered to selectively deliver the antigen to desired cell types. Also provided is a yeast vehicle comprising a yeast strain capable of producing a heterologous precursor protein having a dibasic amino acid processing site. Such a yeast strain is capable of correctly processing the precursor protein into at least one cleavage product protein.

In some examples, the mutant polypeptide is encoded by an oncogene, such as for example, ras, or encoded by a virus, such as for example, HIV, HCV or HBV. In some examples the mutant polypeptide is a tumor-associated antigen or a protein expressed by cancer cells.

### Preparation of Vectors

Provided herein are compositions comprising a vector, such as a yeast vehicle, in association with a mutant polypeptide. Such association includes expression of the polypeptide by the vector, such as for example, by a recombinant yeast, introduction of a mutant polypeptide into a vector, physical attachment of the mutant polypeptide to the vector, and mixing of the vector and mutant polypeptide together, such as in a buffer or other solution for formulation. Such methods are deemed routine for the skilled artisan.

By way of illustration, a yeast vector is described below. In some examples, a yeast cell used to prepare the yeast vehicle is transformed with a heterologous nucleic acid molecule encoding a mutant polypeptide such that the polypeptide is expressed by the yeast cell. Such a yeast is also referred to herein as a recombinant yeast or a recombinant yeast vehicle. The yeast cell can then be loaded into a dendritic cell as an intact cell, the yeast cell can be killed, or it can be derivatized such as by formation of yeast spheroplasts, cytoplasts, ghosts, or subcellular particles, any of which is followed by loading of the derivative into the dendritic cell. Yeast spheroplasts can also be directly transfected with a recombinant nucleic acid molecule (e.g., the spheroplast is produced from a whole yeast, and then transfected) in order to produce a recombinant spheroplast that expresses an antigen.

According to the present invention, an isolated nucleic acid molecule, or nucleic acid sequence, is a nucleic acid molecule or sequence that has been removed from at least one component with which it is naturally associated. As such, "isolated" does not necessarily reflect the extent to which the nucleic acid molecule has been purified. An isolated nucleic acid molecule useful for transfecting a vector, such as a yeast vehicles, includes DNA, RNA, or derivatives of either DNA or RNA. An isolated nucleic acid molecule can be double stranded or single stranded. An isolated nucleic acid molecule useful in the present invention includes nucleic acid molecules that encode a protein or a fragment thereof, as long as the fragment contains at least one epitope useful in a composition of the present invention.

Nucleic acid molecules can be transformed into a vector, such as a yeast vehicle, by any method known in the art, including, but not limited to, diffusion, active transport, liposome fusion, electroporation, bath sonication, and genetic engineering.

Nucleic acid molecules transformed into yeast vehicles can include nucleic acid sequences encoding one or more mutant polypeptides. Such nucleic acid molecules can comprise partial or entire coding regions, regulatory regions, or combinations thereof. One advantage of yeast strains is their ability to carry a number of nucleic acid molecules and of being capable of producing a number of heterologous proteins. In some examples, a number of antigens to be produced by a yeast vehicle is any number of antigens that can be reasonably produced by a yeast vehicle, and typically ranges from at least one to at least about 5 or more, with from about 2 to about 5 antigens.

A mutant polypeptide encoded by a nucleic acid molecule within a yeast vehicle can be a full-length protein, or can be a functionally equivalent protein in which amino acids have been deleted (e.g., a truncated version of the protein), inserted, inverted, substituted and/or derivatized (e.g., acetylated, glycosylated, phosphorylated, tethered by a glycerophosphatidyl inositol (GPI) anchor) such that the modified protein has a biological function substantially similar to that of the natural protein (or which has enhanced or inhibited function as compared to the natural protein, if desired). Modifications can be accomplished by techniques known in the art including, but not limited to, direct modifications to the protein or modifications to the nucleic acid sequence encoding the protein using, for example, classic or recombinant DNA techniques to effect random or targeted mutagenesis.

Expression of mutant polypeptides in vectors is accomplished using techniques known to those skilled in the art. Briefly, a nucleic acid molecule encoding at least one desired mutant polypeptide is inserted into an expression vector in such a manner that the nucleic acid molecule is operatively linked to a transcription control sequence in order to be capable of effecting either constitutive or regulated expression of the nucleic acid molecule when transformed into a host yeast cell. Nucleic acid molecules encoding one or more mutant polypeptides can be on one or more expression vectors operatively linked to one or more transcription control sequences.

In a recombinant molecule of the present invention, nucleic acid molecules are operatively linked to expression vectors containing regulatory sequences such as transcription control sequences, translation control sequences, origins of replication, and other regulatory sequences that are compatible with the vector and that control the expression of nucleic acid molecules. In particular, recombinant molecules of the present invention include nucleic acid molecules that are operatively linked to one or more transcription control sequences. The phrase "operatively linked" refers to linking a nucleic acid molecule to a transcription control sequence in a manner such that the molecule is able to be expressed when transfected (i.e., transformed, transduced or transfected) into a host cell.

Transcription control sequences, which can control the amount of protein produced, include sequences which control the initiation, elongation, and termination of transcription. Particularly important transcription control sequences are those which control transcription initiation, such as promoter and upstream activation sequences. A number of upstream activation sequences (UASs), also referred to as enhancers, are known and can be used in vectors.

Transfection of a nucleic acid molecule into a vector can be accomplished by any method by which a nucleic acid molecule is administered into the cell and includes, but is not limited to, diffusion, active transport, bath sonication, electroporation, microinjection, lipofection, adsorption, and protoplast fusion. Transfected nucleic acid molecules can be integrated into a chromosome or maintained on extrachromosomal vectors using techniques known to those skilled in the art. In the case of yeast, yeast cytoplasts, yeast ghosts, and subcellular yeast membrane extracts or fractions thereof can also be produced recombinantly by transfecting intact yeast microorganisms or yeast spheroplasts with desired nucleic acid molecules, producing the antigen therein, and then further manipulating the microorganisms or spheroplasts using techniques known to those skilled in the art to produce cytoptasts, ghosts or subcellular yeast membrane extracts or fractions thereof containing desired antigens.

Effective conditions for the production of recombinant vectors and expression of the mutant polypeptide by the vector include an effective medium in which the vector can be cultured. An effective medium is typically an aqueous medium comprising assimilable, carbohydrate, nitrogen and phosphate sources, as well as appropriate salts, minerals, metals and other nutrients, such as vitamins and growth factors. The medium may comprise complex nutrients or may be a defined minimal medium. Vectors of the present invention can be cultured in a variety of containers, including, but not limited to, bioreactors, Erlenmeyer flasks, test tubes, microtiter dishes, and petri plates. Culturing is carried out at a temperature, pH and oxygen content appropriate for the yeast strain. Such culturing conditions are well within the expertise of one of ordinary skill in the art (see, for example, Guthrie et al. (eds.), 1991, Methods in Enzymology, vol. 194, Academic Press, San Diego).

In one example of the present invention, as an alternative to expression of a mutant polypeptide in a vector, a vector, such as a yeast vehicle is loaded intracellularly with the mutant polypeptide. Subsequently, the vector, which now contains the mutant polypeptide intracellularly, can be administered to the patient or loaded into a carrier such as a dendritic cell (as described below). With respect to yeast vehicles, mutant polypeptides can be inserted directly into yeast vehicles of the present invention by techniques known to those skilled in the art, such as by diffusion, active transport, liposome fusion, electroporation, phagocytosis, freeze-thaw cycles and bath sonication.

Yeast vehicles that can be directly loaded with a mutant polypeptide include intact yeast, as well as spheroplasts, ghosts or cytoplasts, which can be loaded with antigens after production, but before loading into dendritic cells. Alternatively, intact yeast can be loaded with the antigen, and then spheroplasts, ghosts, cytoplasts, or subcellular particles can be prepared therefrom. Any number of antigens can be loaded into a yeast vehicle, from at least 1, 2, 3, 4 or any whole integer up to hundreds or thousands of antigens, such as would be provided by the loading of a microorganism, by the loading of a mammalian tumor cell, or portions thereof, for example.

In another example, a mutant antigen is physically attached to the vector, such as a yeast vehicle. Physical attachment of the mutant polypeptide to the vector can be accomplished by any method suitable in the art, including covalent and non-covalent association methods which include, but are not limited to, chemically cross-linking the mutant polypeptide to the outer surface of the vector, or biologically linking the mutant polypeptide to the outer surface of the vector, such as by using an antibody or other binding partner. Chemical cross-linking can be achieved, for example, by methods including glutaraldehyde linkage, photoaffinity labeling, treatment with carbodiimides, treatment with chemicals capable of linking di-sulfide bonds, and treatment with other cross-linking chemicals standard in the art. Alternatively, in the case of yeast, a chemical can be contacted with the yeast vehicle that alters the charge of the lipid bilayer of yeast membrane or the composition of the cell wall so that the outer surface of the yeast is more likely to fuse or bind to antigens having particular charge characteristics. Targeting agents such as antibodies, binding peptides, soluble receptors, and other ligands may also be incorporated into a mutant antigen as a fusion protein or otherwise associated with an antigen for binding of the antigen to the vector.

In yet another example, the vector and mutant polypeptide are associated with each other by a more passive, non-specific or non-covalent binding mechanism, such as by gently mixing the vector and the antigen together in a buffer or other suitable formulations.

In some examples of the invention, a vector and mutant antigen are both loaded intracellularly into a carrier such as a dendritic cell or macrophage to form an immunogenic composition. A dendritic cell can be any dendritic cell known in the art. Dendritic cells are cells of monocyte and lymphocyte lineages, and are known to be the most potent antigen presenting cell (APC) and to stimulate antigen-specific T cell responses. Mature dendritic cells are typically identified as having the following cell surface marker phenotype: MAC3⁻, CD80⁺, CD86⁺, CD401°w, CD54⁺, MHC Class I and MHC Class II, and are capable of FITC-dextran uptake. The dendritic cell used in the composition of the present invention is in some examples, isolated from a patient to which the composition is to be administered (i.e., autologous cells). Dendritic cells can be isolated from the bone marrow or peripheral blood. Such cells can be generated, for example, from peripheral blood monocytes by culture in the presence of granulocyte macrophage colony-stimulating factor, IL-4, and TNF-α, for example. Other methods for isolating and generating dendritic cells are known in the art. (See, for example, Wilson et al., 1999, Immunol 162 : 3070-8 ; Romani et al., 1994, J. Exp Med 180 : 83-93; Cauxetal., 1996, J. Exp Med 184 : 695-706; and Kiertscher et al., 1996, J. Leukoc. Biol 59: 208-18).

In order for dendritic cells to efficiently present antigens to native T cells, immature dendritic cells must be activated to mature, as defined by the upregulation of MHC and costimulatory molecules. Yeast provides a powerful activation stimulus to dendritic cells, through Toll-like receptors (TLRs) (see for example, Takeda K. and Akira S., 2005, International Immunology, vol. 17; pages 1-14), mannan, glucan and dectin receptors, resulting in upregulation of co-stimulatory immune receptors, MHC molecules, and secretion of immunomodulatory cytokines. Furthermore, when the yeast is preloaded with the antigen before being loaded to the dendritic cells, it provides antigen to dendritic cells in discrete, concentrated packages that are avidly internalized, thereby effectively increasing the amount of antigen available for processing. As will be appreciated by the skilled artisan, additional vectors can be used to load dendritic cells.

Various forms in which the loading of both components can be accomplished are discussed in more detail below. As used herein, the term "loaded" and derivatives thereof refer to the insertion, introduction, or entry of a component (e.g., the yeast vehicle and/or antigen) into a cell (e.g., a dendritic cell). To load a component intracellularly refers to the insertion or introduction of the component to an intracellular compartment of the cell (e.g., through the plasma membrane and at a minimum, into the cytoplasm, a phagosome, a lysosome, or some intracellular space of the cell). To load a component into a cell references any technique by which the component is either forced to enter the cell (e.g., by electroporation) or is placed in an environment (e.g., in contact with or near to a cell) where the component will be substantially likely to enter the cell by some process (e.g., phagocytosis). Loading techniques include, but are not limited to, diffusion, active transport, liposome fusion, electroporation, phagocytosis, and bath sonication. In some examples, passive mechanisms for loading a dendritic cell with the yeast vehicle and/or antigen are used, such passive mechanisms include phagocytosis of the yeast vehicle and/or antigen by the dendritic cell.

In the case of yeast, the yeast vehicle and the mutant polypeptide can be loaded into the dendritic cell at approximately the same time or simultaneously, although it is also possible to load one component into the cell, followed by the other at some period later. In some examples, the yeast vehicle and the mutant polypeptide are associated with one another prior to loading into the dendritic cell. For example, a recombinant yeast vehicle expressing a mutant polypeptide or any other complex or mixture of yeast vehicle and mutant polypeptide can be loaded into a dendritic cell. The dendritic cell may additionally be loaded with free mutant polypeptide, i.e., a polypeptide that is not directly associated with a yeast vehicle when it is introduced (loaded) into the dendritic cell. The addition of free polypeptide with a yeast vehicle-antigen complex can provide an additional enhancement of the immune response against the polypeptide. The free polypeptide(s) loaded into the dendritic cell does not need to be the same as is expressed by the yeast vehicle, loaded into the yeast vehicle, or otherwise associated with the yeast vehicle. In this manner, the immune response against a target cell or virus can be enhanced.

In some examples, a composition comprising the mutant polypeptide or nucleic acid encoding it, comprises one or more adjuvants, including those as described herein, and/or carriers, although this is not required. Adjuvants are typically substances that generally enhance the immune response of an animal to a specific antigen. Suitable adjuvants include, but are not limited to, TLR agonists as described herein, CpG sequences (see for example, Krieg et al. WO 96/02555), single stranded RNA, double stranded RNA, Freund's adjuvant, other bacterial cell wall components (including LPS, flagellin), aluminum-based salts, calcium-based salts, silica, polynucleotides, toxoids, serum proteins, viral coat proteins, other bacterial-derived preparations, gamma interferon, block copolymer adjuvants, such as Hunter's Titermax adjuvant (CytRx.TM., Inc. Norcross, Ga.), Ribi adjuvants (available from Ribi ImmunoChem Research, Inc., Hamilton, Mont.), and saponins and their derivatives, such as Quil A (available from Superfos Biosector A/S, Denmark).

Carriers are typically compounds that increase the half-life of a therapeutic composition in the treated animal. Suitable carriers include, but are not limited to, polymeric controlled release formulations, biodegradable implants, liposomes, oils, esters, and glycols.

Immunogenic compositions of the present invention may also comprise one or more pharmaceutically acceptable excipients. As used herein, a "pharmaceutically acceptable excipient" refers to any substance suitable for delivering a composition useful in the methods of the present invention to a suitable *in vivo* or *ex vivo* site. In some examples, pharmaceutically acceptable excipients are capable of maintaining a vector (or a dendritic cell comprising the vector) in a form that, upon arrival of the vector or cell at a target cell, tissue, or site in the body, the vector (associated with a mutant polypeptide) or the dendritic cell (loaded with a vector and mutant antigen), is capable of eliciting an immune response, including a cellular immune response, a humoral immune response, or both, at the target site (noting that the target site can be systemic). Suitable excipients of the present invention include excipients or formularies that transport, but do not specifically target the composition or vaccine to a site (also referred to herein as non-targeting carriers). Examples of pharmaceutically acceptable excipients include, but are not limited to water, saline, phosphate buffered saline, Ringer's solution, dextrose solution, serum-containing solutions, Hank's solution, other aqueous physiologically balanced solutions, oils, esters and glycols. Aqueous carriers can contain suitable auxiliary substances required to approximate the physiological conditions of the recipient, for example, by enhancing chemical stability and isotonicity. Suitable auxiliary substances include, for example, sodium acetate, sodium chloride, sodium lactate, potassium chloride, calcium chloride, and other substances used to produce phosphate buffer, Tris buffer, and bicarbonate buffer. Auxiliary substances can also include preservatives, such as thimerosal, m- or o-cresol, formalin and benzol alcohol.

### Cancer

As used herein, cancer includes any type of tumor or neoplasia, including, but not limited to, colorectal cancer, melanomas, squamous cell carcinoma, breast cancers, head and neck carcinomas, thyroid carcinomas, soft tissue sarcomas, bone sarcomas, testicular cancers, prostatic cancers, ovarian cancers, bladder cancers, skin cancers, brain cancers, angiosarcomas, hemangiosarcomas, mast cell tumors, primary hepatic cancers, lung cancers, pancreatic cancers, gastrointestinal cancers, renal cell carcinomas, hematopoietic neoplasias and metastatic cancers thereof. Examples of specific cancer antigens include, but are not limited to, MAGE (including but not limited to MAGE3, MAGEA6, MAGEA10), NY-ESO-1, gp100, tyrosinase, EGFR, PSA, PSMA, VEG-F, PDGFR; KIT, PMSA, CEA, HER2/neu, Muse-1, hTERT, MART1, TRP-1, TRP-2, Bcr-Abl, and mutant oncogenic forms of p53 (TP53), p73, Ras, PTENSrc, p38, BRAF, APC (adenomatous polyposis coli), myc, VHL (von Hippel Lindau protein), Rb-1 (retinoblastoma), Rb-2, BRCA1, BRCA2, AR (androgen receptor), Smad4, MDR1 and FLT3.

In some examples, a cancer antigen is, or is obtainable from, a molecule (such as a protein, a peptide, a glycoprotein, or a carbohydrate) that is suitable for targeting by a therapeutic and/or prophylactic agent. Molecular targets for therapeutic and/or prophylactic cancer agents are known in the art, and include, but are not limited to, cell surface receptors (such as receptor tyrosine phosphatases, receptor serine/threonine kinases, and receptor tyrosine kinases), intracellular signaling molecules (such as intracellular tyrosine kinases and other secondary signaling molecules), and transcription factors, cell cycle regulators, proteasome components, proteins involved in angiogenesis, and proteins involved in apoptosis control. Targeting therapeutic and/or prophylactic agents to cancer has been observed to result in the presence of escape mutants, that is, mutant polypeptides. For example, mutations were found in Bcr-Abl that were reported to make individuals previously responsive with treatment of the Bcr-Abl tyrosine kinase inhibitor imatinib (Gleevec) become resistant to the treatment. Gorre et al., Science 2001, 293:876-880; Shah et al., Cancer Cell 2002, 2:117-125; Branford et al., Blood 2002, 99(9).3742-3745; Deininger et al., Blood 2005, 105(7):2640-263. Similarly, mutations in EGFR, have also been found in non-small cell lung cancer (NSCLC) patients that make them resistant to gefitinib (Iressa) or erlotinib (Tarceva) treatment. Kobayashi et al., N. Engl. J Med., 2005, 352(8):786-792. The effectiveness of these anti-cancer agents are therefore significantly limited by the emergence of mutant polypeptides.

Accordingly, provided herein are immunogenic compositions comprising mutant polypeptides encoded by oncogenes and/or expressed by cancer cells (or mimetopes thereof), or nucleic acid encoding the mutant polypeptides that are known to emerge or which have emerged with a specific mutation in response to administration of a therapeutic and/or prophylactic agent, as well as methods for eliciting an immune response to the mutant polypeptide or cell expressing the mutant polypeptide. In some examples, the immune response is a cellular immune response. In some examples, the immune response is a humoral immune response. In other examples, the immune response includes both cellular and humoral responses.

Polypeptide mutants of cancer antigens can be preexisting in the mammal, i.e., present at the time of the diagnosis, and selectively emerge as a result of administration of the therapeutic and/or prophylactic agent(s). Alternatively, the polypeptide mutants can emerge as a result of the pressure imposed by the agent. The mutation can be located at any amino acid position in the cancer antigen. Although the mutations of polypeptides are described in the context of a single mutation, it is to be understood that the mutant polypeptide can comprise more than one (such as two, three, four, five, or more) amino acid mutations. For example, the cancer antigen may contain one or more mutations at different amino acid positions. The cancer antigen may further contain other mutations, such as mutations associated with transforming events. Illustrative examples of mutant polypeptides of cancer antigens are disclosed in Table I.

**Table I**

| **No.** | **Escape mutants** | **therapeutic agent** | **Target** | **Source** | **Reference** |
|---|---|---|---|---|---|
| 1. | M244V | Gleevec | Bcr-Abl* | CML patient | Deininger et al. |
| 2. | L248R | Gleevec | Bcr-Ab1 | CML patient | Deininger et al. |
| 3. | G250E | Gleevec | Ber-Abl | CML patient | Deininger et al. |
| 4. | G250A | Gleevec | Ber-Abl | CML patient | Deininger et al. |
| 5. | Q252H | Gleevec | Ber-Abl | CML patient | Deininger et al. |
| 6. | Q252R | Gleevec | Bcr-Abl | CML patient | Deininger et al. |
| 7. | Y253F | Gleevec | Bcr-Abl | CML patient | Deininger et al. |
| 8. | Y253H | Gleevec | Ber-Abl | CML patient | Deininger et al. |
| 9. | E255K | Gleevec | Bcr-Ab1 | CML patient | Deininger et al. |
| 10. | E255V | Gleevec | Bcr-Abl | CML patient | Deininger et al. |
| 11. | D276G | Gleevec | Bcr-Abl | CML patient | Deininger et al. |
| 12. | F311L | Gleevec | Bcr-Abl | CML patient | Deininger et al. |
| 13. | T3151 | Gleevec | Bcr-Abl | CML patient | Deininger et al. |
| 14. | T315N | Gleevec | Bcr-Abl | CML patient | Deininger et al. |
| 15. | F317L | Gleevec | Bcr-Abl | CML patient | Deininger et al. |
| 16. | M343T | Gleevec | Bcr-Abl | CML patient | Deininger et al. |
| 17. | M351T | Gleevec | Bcr-Abl | CML patient | Deininger et al. |
| 18. | E355G | Gleevec | Bcr-Abl | CML patient | Deininger et al. |
| 19. | F359A | Gleevec | Bcr-Abl | CML patient | Deininger et al. |
| 20. | F359V | Gleevec | Bcr-Abl | CML patient | Deininger et al. |
| 21. | V379I | Gleevec | Bcr-Abl | CML patient | Deininger et al. |
| 22. | F382L | Gleevec | Bcr-Abl | CML patient | Deininger et al. |
| 23. | L387M | Gleevec | Bcr-Abl | CML patient | Deininger et al. |
| 24. | H396P | Gleevec | Bcr-Abl | CML patient | Deininger et al. |
| 25. | H396R | Gleevec | Bcr-Abl | CML patient | Deininger et al. |
| 26. | S417Y | Gleevec | Bcr-Abl | CML patient | Deininger et al. |
| 27. | E459K | Gleevec | Bcr-Abl | CML patient | Deininger et al. |
| 28. | F486S | Gleevec | Bcr-Abl | CML patient | Deininger et al. |
| 29. | L248V | Gleevec | Bcr-Abl | *In vitro* | Azam et al. (2003) |
| 30. | G250R | Gleevec | Bcr-Abl | In vitro | Azam et al. (2003) |
| 31. | Y253C | Gleevec | Bcr-Abl | *In vitro* | Azam et al (2003) |
| 32. | Y257C | Gleevec | Bcr-Abl | *In vitro* | Azam et al. (2003) |
| 33. | E258D | Gleevec | Bcr-Abl | *In nitro* | Azam et al. (2003) |
| 34. | S265T | Gleevec | Bcr-Abl | *In vitro* | Azam et al. (2003) |
| 35. | S265I | Gleevec | Bcr-Abl | *In vitro* | Azam et al. (2003) |
| 36. | L266M | Gleevec | Bcr-Abl | *In vitro* | Azam et al. (2003) |
| 37. | L266V | Olcevec | Ber-Abl | *In vitro* | Azam et al. (2003) |
| 38. | V268/270A | Gleevee | Bcr-Abl | *In vitro* | Azam et al. (2003) |
| 39. | A269V | Gleevec | Bcr-Abl | *In vitro* | Adam et al. (2003) |
| 40. | E275K | Gleevec | Bcr-Abl | *In vitro* | Azam et all. (2003) |
| 41. | D276V | Gleevec | Ber-Abl | *In vitro* | Azam et al. (2003) |
| 42. | M278L | Gleevec | Bcr-Abl | *In vitro* | Azam et al. (2003) |
| 43. | E279K | Gleevec | Bcr-Abl | *In vitro* | Azam et all. (2003) |
| 44. | E281K | Gleevec | Bcr-Abl | *In vitro* | Azam et al. (2003) |
| 45. | E282D | Gleevec | Bcr-Abl | *In vitro* | Adam et al. (2003) |
| 46. | F283L | Gleevec | Bcr-Abl | *In vitro* | Azam et al. (2003) |
| 47. | L284F | Gleevec | Bcr-Abl | *In vitro* | Azam et al. (2003) |
| 48. | V289S | Gleevec | Bcr-Abl | *In vitro* | Azam et al. (2003) |
| 49. | M290L | Gleevec | Bcr-Abl | *In vitro* | Azam et al. (2003) |
| 50. | M290T | Gleevec | Bcr-Abl | *In vitro* | Azam et al. (2003) |
| 51. | K291E | Gleevec | Bcr-Abl | *In vitro* | Azam et al. (2003) |
| 52. | K291R | Gleevec | Bcr-Abl | *In vitro* | Azam et al. (2003) |
| 53. | E292Q | Gleevec | Bcr-Abl | *In vitro* | Azam et al. (2003) |
| 54. | K294R | Gleevec | Bcr-Abl | *In vitro* | Azam et al. (2003) |
| 55. | Q300H | Gleevec | Bcr-Abl | *In vitro* | Azam et al. (2003) |
| 56. | L301F | Gleevec | Bcr-Abl | *In vitro* | Azam et al. (2003) |
| 57. | F311V | Gleevec | Ber-Abl | *In vitro* | Azam et al. (2003) |
| 58. | T315S | Gleevec | Bar-abel | *In nitro* | Azam et al. (2003) |
| 59. | T315G | Gleevec | Bcr-Abl | *In vitro* | Azam et all. (2003) |
| 60. | E316D | Gleevec | Bcr-Abl | *In vitro* | Azam et al. (2003) |
| 61. | G321W | Gleevec | Bcr-Abl | *In vztro* | Azam et al. (2003) |
| 62. | N3318 | Gleevec | Bcr- Abl | *In vitro* | Azam et al. (2003) |
| 63. | V338G | Gleevec | Ber-Abl | *In vitro* | Azam et al. (2003) |
| 64. | V339A | Gleevec | Bcr-Abl | *In vitro* | Azam et al. (2003) |
| 65. | V339G | Gleevec | Bcr-Abl | *In vitro* | Azam et al. (2003) |
| 66. | A344V | Gleevec | Bcr-Abl | *In vitro* | Azam et al. (2003) |
| 67. | Q346H | Gleevec | Bcr-Abl | *In vitro* | Azam et al. (2003) |
| 68. | M351I | Gleevee | Bcr-Abl | *In vitro* | Azam et al. (2003) |
| 69. | E352K. | Gleevec | Bcr-Abl | *In vitro* | Azam et al. (2003) |
| 70. | I360F | Gleevec | Bcr-Abl | *In vitro* | Azam et a1. (2003) |
| 71. | A366D | Gleevec | Bcr-Abl | *In vitro* | Adam et al. (2003) |
| 72. | G372R | Gleevec | Bcr-Abl | *In vitro* | Azam et al. (2003) |
| 73. | E373K | Gleevec | Bcr-Abl | *In vitro* | Azam et al. (2003) |
| 74. | V379E | Gleevec | Bcr-Abl | *In vitro* | Azam et al. (2003) |
| 75. | V379A | Gleevec | Bcr-Abl | *In vitro* | Azam et al. (2003) |
| 76. | L384M | Gleevec | Bcr-Abl | *In vitro* | Azam et al. (2003) |
| 77. | M388I | Gleevec | Bcr-Abl | *In vitro* | Azam et al. (2003) |
| 78. | G398R | Gleevec | Bcr-Abl | *In vitro* | Azam et al. (2003) |
| 79. | Y440C | Gleevec | Bcr-Abl | *In vitro* | Azam et al. (2003) |
| 80. | E450K | Gleevec | Bcr-Abl | *In vitro* | Azam et al. (2003) |
| 81. | L451M | Gleevec | Bcr-Abl | *In vitro* | Azam et al. (2003) |
| 82. | G463D | Gleevec | Bcr- Abl | *In vitro* | Azam et al. (2003) |
| 83. | M472I | Gleevec | Bcr-Abl | *In vitro* | Azam et al. (2003) . |
| 84. | R473L | Gleevec | Bcr-Abl | *In vitro* | Azam et al. (2003) |
| 85. | P486S | Gleevec | Bcr-Abl | *In vitro* | Azam et al. (2003) |
| 86. | E494A | Gleevec, | Bcr-Abl | *In vitro* | Azam et al. (2003) |
| 87. | E499K | Gleevec | Bcr-Abl | *In vitro* | Azam et al. (2003) |
| 88. | E499I | Gleevec | Bcr-Abl | *In vitro* | Azam et al. (2003) |
| 89. | I502M | Gleevec | Bcr-Abl | *In vitro* | Azam et al. (2003) |
| 90. | E509D | Gleevec | Bcr-Abl | *In vitro* | Azam et al. (2003) |
| 91. | E38K | Gleevec | Bcr-Abl | *In vitro* | Azam et al. (2003) |
| 92. | A45G | Gleevec | Bcr-Abl | *In vitro* | Azam et al. (2003) |
| 93. | A45V | Gleevec | Bcr-Abl | *In vitro* | Azam et al. (2003) |
| 94. | K51Q | Gleevec | Bcr-Abl | *In vitro* | Azam et al. (2003) |
| 95. | E52K | Gleevec | Bcr-Abl | *In vitro* | Azam et al. (2003) |
| 96. | N53D | Gleevec | Bcr-Abl | *In vitro* | Azam et al. (2003) |
| 97. | N53T | Gleevec | Bcr-Abl | *In vitro* | Azam et al. (2003) |
| 98. | A56V | Gleevec | Bcr-Abl | *In vitro* | Azam et al. (2003) |
| 99. | G57E | Gleevec | Bcr-Abl | *In vitro* | Azam et al. (2003) |
| 100. | P58R | Gleevec | Bcr-Abl | *In vitro* | Azam et al. (2003) |
| 101. | E60K | Gleevec | Bcr-Abl | *In vitro* | Azam et al. (2003) |
| 102. | N64Y | Gleevec | Ber-Abl | *In vitro* | Azam et al. (2003) |
| 103. | V67G | Gleevec | Bcr-Abl | *In vitro* | Azam et al. (2003) |
| 104. | A68E | Gleevec | Bcr-Abl | *In vitro* | Azam et al. (2003) |
| 105. | A68V | Gleevec | Bcr-Abl | *In vitro* | Azam et al. (2003) |
| 106. | Y70D | Gleevec | Bcr-Abl | *In vitro* | Azam et al. (2003) |
| 107. | Y70H | Gleevec | Bcr-Abl | *In vitro* | Azam et al. (2003) |
| 108. | Y70N | Gleevec | Bcr-Abl | *In vitro* | Azam et al. (2003) |
| 109. | D71N | Gleevec | Bcr-Abl | *In vitro* | Azam et al. (2003) |
| 110. | W110C | Gleevec | Bcr-Abl | *In vitro* | Azam et al. (2003) |
| 111. | V111L | Gleevec | Bcr-Abl | *In vitro* | Azam et al. (2003) |
| 112. | P112Q | Gleevec | Bcr-Abl | *In vitro* | Adam et al. (2003) |
| 113. | S113N | Gleevec | Bcr-Abl | *In vitro* | Azam et al. (2003) |
| 114. | V119G | Gleevec | Bcr-Abl | *In vitro* | Azam et al.(2003) |
| 115. | S12IR | Gleevec | Ber-Abl | *In vitro* | Azam et al. (2003) |
| 116. | S148C | Gleevec | Bcr-Abl | *In vitro* | Azam et al. (2003) |
| 117. | S157T | Gleevec | Ber-Abl | *In vitro* | Azam et al. (2003) |
| 118. | S187F | Gleevec | Bcr-Abl | *In vitro* | Azam et al. (2003) |
| 119. | S187P | Gleevec | Ber-Abl | *In vitro* | Azam et al. (2003) |
| 120. | V205E | Gleevec | Ber-Abl | *In vitro* | Azam et al. (2003) |
| 121. | A217G | Gleevec | Bcr-Abl | *In vitro* | Azam et al. (2003) |
| 122. | K219E | Gleevec | Bcr-Abl | *In vitro* | Azam et al. (2003) |
| 123. | T224A | Gleevec | Bcr-Abl | *In vitro* | Azam et al. (2003) |
| 124. | P230L | Gleevec | Bcr-Abl | *In vitro* | Azam et al. (2003) |
| 125. | V256A | Gleevec | Bcr-Abl | *In vitro* | Azam et al, (2003) |
| 126. | K271N | Gleevec | Bcr-Abl | *In vitro* | In Azam et al. (2003) |
| 127. | E286K | Gleevec | Bcr-Abl | *In vitro* | Azam et al.(2003) |
| 128. | E286V | Gleevec | Ber-Abl | *In vitro* | Azam et al. (2003) |
| 129. | I313A | Gleevec | Bcr-Abl | *In vitro* | Azam et al. (2003) |
| 130. | M318A | Gleevec | Bcr-Abl | *In vitro* | Azam et al. (2003) |
| 131. | I360F | Gleevec | Ber-Abl | *In vitro* | Azam et al. (2003) |
| 132. | F382A | Gleevec | Ber-Abl | *In vitro* | Azam et al. (2003) |
| 133. | T6071 | Gleevec | KIT | GIST patients | Tamborini et al. |
| 134. | T6811 | Gleevec | PDGFR | Patient with Idiopathic hypereosinophilic syndrome | Cools et al. (2003) |
| 135. | T790M | Iressa or Tarceva | EGFR | NSCLC patient | Kobayashi et al. |
| 136. | T106M | SB 203580 | p38 | *In vitro* | Eyers et al.** |
| 137. | T341M | Tyrosine kinase inhibitors | Src | *In vitro* | Blencke et al. (2004) |
| 138. | V561M | Tyrosine kinase inhibitors | FGFR | *In vitro* | Blencke et al. (2004) |
| 139. | A627T | Kinase inhibitors | FLT3 | *In vitro* | Cools et al. (2004) |
| 140. | N676D | Kinase inhibitors | FLT3 | *In vitro* | Cools et al. (2004) |
| 141. | N676S | Kinase inhibitors | FLT3 | *In vitro* | Cools et al. (2004) |
| 142. | F691L | Kinase inhibitors | FLT3 | *In vitro* | Cools et al. (2004) |
| 143. | F691I | Kinase inhibitors | FLT3 | *In vitro* | Cools metal. (2004) |
| 144. | G697R | Kinase inhibitor | FLT3 | *In vitro* | Cools et al. (2004) |
| 145. | G697S | Kinase inhibitors | FLT3 | *In vitro* | Cools et al. (2004) |

| | | | | | |
|---|---|---|---|---|---|
| *wild-type Abl numbering according to ABL exon la. **Eyers et al., *Chem. Biol.* 1998,5,321-328. | | | | | |

In some examples, a polypeptide mutant can be a fusion polypeptide that contains multiple immunogenic domains from one or more mutant polypeptides of cancer antigens. For example, it is known that there are several different mutations in the Bcr-Abl protein that emerge upon administration of Gleevee. A mutant polypeptide may comprise one or more Bcr-Abl mutations at the same position and/or different positions and/or combinations of mutations at more than one position.

In some examples, the polypeptide mutant comprises a mutation in Bcr-Abl. Bcr-Abl is a constitutively active tyrosine kinase that results from a DNA translocation between chromosome 9 and 22. Bcr-Abl is reported to be causal to the pathogenesis of chronic myeloid leukemia (CML), and its constitutive kinase activity central to its capacity to transform hematopoietic cells *in vivo.* Imatinib (Gleevec, 2-phenylaminopyrimidme), a tyrosine kinase inhibitor, is a therapeutic agent for CML. Various escape mutations in Bcr-Abl that render the protein resistant to drug therapy (e.g., Gleevec treatment) have been identified *in vivo* and *in vitro.* Deininger et al., Blood, 2005, 105(7):2640-2653; Azam et al., Cell, 2003,112:831-43. Table I provides an exemplary list of escape mutants of Bcr-Abl (along with other cancer antigens) identified in mammals who develop resistance to treatment of Gleevec (Mutation Nos. 1-28), as well as additional mutations identified by in vitro methods (Mutation Nos. 29-132). These mutations are located in various domains of Bcr-Abl, including, but not limited to, the kinase domain (such as the P-loop, the A-loop, T315, the C-helix, the SH3 contact region, or the SH2 contact region), the cap domain, the SH3 domain, the SH2 domain, and other linker regions.

In some examples, the mutant polypeptide comprises a mutation in EGFR, EGFR is a receptor tyrosine kinase that plays a key role in the initiation of cell division in both normal and cancer cells. In a number of cancers, including non-small cell lung cancer (NSCLC) and glioblastoma (brain cancer), it is reported that EGFR is either overexpressed or mutated, and these changes are believed to be associated with the formation and growth of tumors. Two oral anilinoquinazoline EGFR tyrosine kinase inhibitors, gefitinib (Iressa) and erlotinib (Tarceva), have been approved in the United States for treatment of NSCLC. An escape mutation, T790M, was found in EGFR that is reported to render the mammalian subject resistant to treatment of Iressa or Tarceva.

Accordingly, provided herein are compositions comprising a mutant polypeptide of EGFR (or mimetope thereof) or nucleic acid encoding EGFR and methods for their use in eliciting an immune response. In some examples, the immune response is a cellular immune response. In some examples, the immune response is an humoral immune response. In other examples, the immune response includes both a cellular and humoral immune response. In some examples, the mutant polypeptide comprises a mutation in the kinase domain of EGFR. In some examples, the mutant polypeptide comprises the T790M mutation with respect to the wild-type EGFR polypeptide.

In some examples, the mutant polypeptide comprises a mutation in platelet-derived growth factor receptor (PDGFR). PDGFR, is a receptor tyrosine kinase. Activation of PDGFR is reported to be critical for the progression of various types of cancers such as glioblastoma, dermatofibrosarcoma protuberans, and CML. A single escape mutation (T674I) has been identified in a patient with hypereosinophilic syndrome treated with Gleevec. (Cools et al., New Engl. J. Med., 2003, 348:1201-1214.) Accordingly, provided herein are compositions comprising a mutant polypeptide of PDGFR (or mimetope thereof) or nucleic acid encoding PDGFR and methods for their use in eliciting an immune response. In some examples, the immune response is a cellular immune response. In some examples, the immune response is an humoral immune response. In other examples, the immune response includes both a cellular and humoral immune response. In some examples, the mutant polypeptide comprises a mutation in the kinase domain of PDGFR. In some examples, the mutant polypeptide comprises the T764I mutation with respect to the wild-type PDGFR polypeptide.

In some embodiments, the mutant polypeptide comprises a mutation of KIT. KIT is a tyrosine kinase receptor for stern-cell factor (SCF). Activation of KIT by mutations in the kinase domain is reported to be associated with gastrointestinal stromal tumor (GIST) and other types of tumors. An escape mutation (T670I) was found in KIT that was reported to render the patient resistant to treatment with Gleevec. (Tamborini et al., Gastroenterology, 2004, 127:294-299.) Accordingly, provided herein are compositions comprising a mutant polypeptide of KIT (or mimetope thereof) or nucleic acid encoding KIT and methods for their use in eliciting an immune response. In some examples, the immune response is a cellular immune response. In some examples, the immune response is an humoral immune response. In other examples, the immune response includes both a cellular and humoral immune response. In some examples, the mutant polypeptide comprises a mutation in the kinase domain of KIT. In some examples, the mutant polypeptide comprises the T670I mutation with respect to the wild-type KIT polypeptide.

In some examples, the mutant polypeptide comprises a mutation in FLT3, that emerges in response to administration of a targeted agent, including amino acid mutations selected from the group consisting of A627T, N676D, N676S, F69IL, F691I, G697R, and G697S, relative to wild-type FLT-3 polypeptide. Cools et al., Cancer Res., 2004, 64:6385-6389. In some examples, the mutant polypeptide comprises a mutation in p38, including a mutation at position T106 relative to the wild-type protein. In other examples, the mutant polypeptide comprises a mutation in Arc, including a mutation at amino acid position T341 relative to the wild-type protein. In some examples, the mutant polypeptide comprises a mutation in FGFR, including an amino acid mutation at position V561 relative to the wild-type protein. Accordingly, provided herein are compositions comprising such mutant polypeptides (or mimetope thereof) or nucleic acid encoding the mutant polypeptides and methods for their use in eliciting an immune response. In some examples, the immune response is a cellular immune response. In some examples, the immune response is an humoral immune response. In other examples, the immune response includes both a cellular and humoral immune response. In some examples, a mutant polypeptide that is known to emerge or that has emerged with a specific mutation in response to an agent is immunogenic on its own, that is, without being associated with an adjuvant but this is not required. In other examples, a mutant polypeptide that is known to emerge or that has emerged in response to an agent is immunogenic in association with an adjuvant, such as a Toll-like receptor ligand or agonist, or CpG nucleotide sequence, or other vector or vehicle, such as a yeast vehicle, that promotes its antigenicity. Accordingly, the compositions described herein are used for eliciting an immune response to a mutant polypeptide in a mammal, comprising administration to the mammal of an effective amount of the composition in conjunction with the targeted therapeutic and/or prophylactic agent. In some examples, the composition comprises one or more of the following:
i) a yeast vehicle comprising nucleic acid which encodes at least one mutant polypeptide, a fragment thereof that comprises a mutation, or a mimetope;
ii) a yeast vehicle comprising at least one mutant polypeptide, a fragment thereof that comprises a mutation, or a mimetope;
iii) a yeast vehicle in association with at least one mutant polypeptide, a fragment thereof that comprises a mutation, or a mimetope;
iv) a yeast vehicle comprising nucleic acid which encodes at least one mutant polypeptide, a fragment thereof that comprises a mutation, or a mimetope loaded intracellularly into a dendritic cell; or
v) a yeast vehicle and at least one mutant polypeptide, a fragment thereof that comprises a mutation, or a mimetope loaded intracellularly into a dendritic cell,
wherein the mutant polypeptide is known to emerge or has emerged with at least one specific mutation in response to administration of a targeted therapeutic and/or prophylactic agent.

Further the compositions may be used in preparation of or manufacture of medicaments for eliciting an immune response to a mutant polypeptide in a mammal in conjunction with a targeted therapeutic and/or prophylactic agent. In some examples, the mutant polypeptide is an oncogene, a tumor-associated antigen or a polypeptide expressed by a cancer cell. In some examples the cancer cell is selected from the group consisting of colorectal cancer, melanomas, squamous cell carcinoma, breast cancers, head and neck carcinomas, thyroid carcinomas, soft tissue sarcomas, bone sarcomas, testicular cancers, prostatic cancers, ovarian cancers, bladder cancers, skin cancers, brain cancers, angiosarcomas, hemangiosarcomas, mast cell tumors, primary hepatic cancers, lung cancers, pancreatic cancers, gastrointestinal cancers, renal cell carcinomas, hematopoietic neoplasias and metastatic cancers.

In some examples the immune response is a cellular immune response. In other examples the immune response is an humoral immune response. In yet other examples the immune response includes both a cellular and humoral immune response.

In addition, the compositions described herein are used to treat a disease in a mammal, comprising administration to the mammal of an effective amount of the composition, wherein the disease is associated with a mutant polypeptide that is known to emerge or has emerged with at least one specific mutation in response to administration of a targeted therapeutic and/or prophylactic agent. In some examples the compositions are used in conjunction with the targeted therapeutic and/or prophylactic agent. Further, the compositions may be used in prepartion or manufacture of medicaments for treating the disease in a mammal in conjunction with a targeted therapeutic and/or prophylactic agent. In some examples, the mutant polypeptide, is an oncogene, a tumor-associated antigen or a polypeptide expressed by a cancer cell. In some examples the cancer cell is selected from the group consisting of colorectal. cancer, melanomas, squamous cell carcinoma, breast cancers, head and neck carcinomas, thyroid carcinomas, soft tissue sarcomas, bone sarcomas, testicular cancers, prostatic cancers, ovarian cancers, bladder cancers, skin cancers, brain cancers, angiosarcomas, hemangiosarcomas, mast cell tumors, primary hepatic cancers, lung cancers, pancreatic cancers, gastrointestinal cancers, renal cell carcinomas, hematopoietic neoplasias and metastatic cancers. In some examples the disease is cancer.

Methods of identifying new mutant polypeptides in a cancer antigen that emerge as a result of administration of agents are known in the art. Escape mutations identified by *in vitro* methods have shown a high degree of correlation with those that develop *in vivo.* (Azam et al, Cell, 2003,112:831-843; Cools et al., Cancer Research, 2004, 64:6385-6389; Blencke et al., Chem. Biol., 2004,11:691-701.) For example, Azam et al. provided a screening method for identifying resistant mutant polypeptides to target-directed anti-cancer agents, which is generally applicable to any agent-mutant polypeptide pair. (Azam et al., Biol. Proced Online, 2003,5(1):204-210.) Briefly, the cDNA encoding the target mutant polypeptide is cloned in a cloning vector and subjected to random mutagenesis, rendering a library of mutations in the target cancer polypeptide. The library is then introduced into cells susceptible to treatment with the agent. Colonies that are resistant to treatment with the agent are then selected in the presence of the therapeutic agent, isolated, and sequenced to reveal the putative mutations. To validate the resistant phenotype of each candidate mutation, mutations can also be created in the native cDNA de novo by site-directed mutagenesis. The mutant cDNAs are introduced into drug-sensitive cells to confirm their drug-resistant phenotypes. Drug resistance can further be confirmed by cell proliferation assays. Mutations can also be analyzed for their structural consequences by mapping into a model of the protein crystal structure.

### Infectious Disease

As used herein infectious disease refers to disease caused by infectious agents such as RNA viruses including, but not limited to, Retroviruses (such as for example, HIV), Flaviviruses (such as for example, HCV), Reoviruses, Picornaviruses, Coronaviruses, Filoviruses, Rhabdoviruses, Bunyaviruses, Orthomyxoviruses, Paramyxoviruses, Arenaviruses. Caliciviruses; and DNA viruses belonging to the families of Hepadnaviruses (such as for example, HBV), Herpesviruses, Poxviruses, Adenoviruses, Parvoviruses, and Papovaviruses. In illustrative examples, the infectious disease is HIV, HBV and HCV. It will be understood by the skilled artisan that the methods as described herein, such as for example, methods for eliciting an immune response to a mutant polypeptide, can be applied to any infectious disease wherein a mutant polypeptide is known to emerge or has emerged in response to a prophylactic and/or therapeutic agent.

### Human Immunodeficiency Virus

HIV is a non-oncogenic retrovirus, specifically a lentivirus, that causes Acquired Immunodeficiency Syndrome (AIDS) in infected individuals. As assessed by the World Health Organization, more than 40 million people are currently infected with HIV and 20 million people have already perished from AIDS. Thus, HIV infection is considered a worldwide pandemic.

There are two currently recognized strains of HIV, HIV-1 and HIV-2. HIV-1 is the principal causes of AIDS around the world. HIV-1 has been classified based on genomic sequence variation into clades. For example, Clade B is the most predominant in North America, Europe, parts of South America and India; Clade C is most predominant in Sub-Saharan Africa; and Clade E is most predominant in southeastern Asia. HIV-1 infection occurs primarily through sexual transmission, transmission from mother to child or exposure to contaminated blood or blood products.

HIV-1 consists of a lipid bilayer envelope surrounding viral structural proteins and an inner core of enzymes and proteins required for viral replication and a genome of two identical linear RNAs. In the viral lipid bilayer viral envelope glycoprotein 41 (gp41) anchors another viral envelope glycoprotein 120 (gp120) that extends from the virus surface and interacts with receptors on the surface of susceptible cells. The HIV-1 genome is approximately 10,000 nucleotides in size and comprises nine genes. It includes three genes common to all retroviruses, the *gag, pol* and *env* genes. The *gag* gene encodes the core structural proteins, the env gene encodes the gp120 and gp41 envelope proteins, and *the pol* gene encodes the viral enzymes reverse transcriptase (RT), integrase and protease (pro). The genome comprises two other genes essential for viral replication, the *tat* gene encoding a viral promoter transactivator and the rev gene which also facilitates gene transcription. Finally, the *nef, vpu, vpr, and vif genes* are unique to lentiviruses and encode polypeptides the functions of which are described in Trono, Cell, 82: 189-192 (1995).

The process by which HIV-1 injects human cells involves interaction of gp120 on the surface of the virus with proteins on the surface of the cells. The common understanding is that the first step in HIV infection is the binding of HIV-1 glycoprotein Env gyp120 to cellular CD4 protein. This interaction causes the viral gap120 to undergo a conformational change and bind to other cell surface proteins, such as CCR5 or CXCR4 proteins, allowing subsequent fusion of the virus with the cell. CD4 has thus been described as the primary receptor for HIV-1 while the other cell surface proteins are described as co-receptors for HIV-1. As used herein, HIV encompasses HIV-1 and HIV-2. Encompassed within the present invention are HIV mutant polypeptides that are known to emerge or that have emerged with a specific mutation in response to an agent, such as a prophylactic and/or therapeutic agent, and include mutations found in any HIV polypeptide, including for example, but not limited to Gag, Env, Pol, and from any HIV clade family, subtypes and/or strains and including, but not limited to, isolates, HIV_{IIIb}, HIV_{SF2}, HIV-1_{SF162}, HIV-1 _{SF170}, HIV_{LAV}, HIV_{LAI}, FRV_{MN}, HIV-1_{CM235}, HIV-1_{US4}, other HIV-1 strains. See, e.g., Myers, et al., Los Alamos Database, Los Alamos National Laboratory, Los Alamos, New Mexico; Myers, et al., Human Retroviruses and Aids, 1990, Los Alamos, New Mexico: Los Alamos National Laboratory. In illustrative examples disclosed herein, mutations of HIV Protease and reverse transcriptase that have emerged in response to agents are disclosed in Table II. In some examples, wherein the agent for use in treating HIV is lamivudine, the mutant polypeptide M194V is specifically excluded. In some examples, a HIV mutant polypeptide that is known to emerge or that has emerged with a specific mutation in response to an agent is immunogenic on its own, that is, without being associated with an adjuvant but this is not required. In other examples, a HIV mutant polypeptide that is known to emerge or that has emerged in response to an agent is immunogenic in association with an adjuvants, as described herein and known in the art, such as, for example, a Toll-like receptor ligand, or CpG nucleotide sequence, or other vector or vehicle, such as a yeast vehicle, that promotes its antigenicity. In some examples, the adjuvant is an amino acid sequence that is expressed as a fusion protein with the mutant polypeptide.

**TABLE II**

| HIV/drug class | Therapeutic Agent | Target | Mutational Escape |
|---|---|---|---|
| Protease Inhibitor | Ritonavir | HIV-1 Protease | V82A/F |
| | | | 154V |
| | | | A71V/T |
| | | | 136L |
| Protease Inhibitor | Altazanavir | HIV-1 Protease | 150L |
| | | | N88S |
| | | | 184V |
| | | | A71V |
| | | | M46I |
| Protease Inhibitor | Saquinavir | HIV-1 Protease | G48V |
| | | | L90M |
| | | | L10I/R/V |
| | | | 154V/L |
| | | | A71V/T |
| | | | G73S |
| | | | V77I |
| | | | V82A |
| | | | 184V |
| NRTI | Lamivudine | HIV-1 Reverse Transcriptase | M184I/V |
| NRTI | Didanosine | HIV-1 Reverse Transcriptase | L74V |
| | | | K65R |
| | | | M184V |
| NNRTI | Emtricitabine | HIV-1 Reverse Transcriptase | M184V/I |

At present, anti-retroviral drug therapy is one means of treating HIV infection or preventing HIV-I transmission from one person to another. At best, even with drug therapy, HW-1 infection is a chronic condition that requires lifelong treatment and there can still be a slow progression to disease. Resistance to drugs against HIV has developed against anti-retroviral drugs of all classes, namely the nucleoside analogue reverse transcriptase inhibitors, the non-nucleoside reverse transcriptase inhibitors, the protease inhibitors and the entry/fusion inhibitors. The number of associated mutations, the degree of increase in resistance caused and the mechanism of resistance differ between many of these drugs.

The mechanisms of drug resistance used by viruses have been studied and characterized. Without being bound by theory, the likelihood that resistant mutants will emerge appears to be a function of at least four factors: 1) the viral mutation frequency; 2) the intrinsic mutability of the viral target site with respect to a specific antiviral; 3) the selective pressure of the antiviral drug; and, 4) the magnitude and rate of virus replication.

With regard to the first factor, for some RNA, viruses, whose polymerase lacks a proofreading mechanism, the mutation frequencies are approximately 3 × 10⁻⁵ /bp/replicative cycle (Holland et al. (1992) Curr. Topics Microbiol Immunol. 176, 1-20; Mansky et al. (1995) J Virol. 69, 5087-94; Coffin (1995) Science 267, 483-489). Thus, a single 10 kb genome, like that of human immunodeficiency virus (HIV), would be expected to contain on average one mutation for every three progeny viral genomes. With calculations of approximately 10¹⁰ new virions being generated daily during HIV infection (Ho et al. (1995) Nature 373, 123-126), a mutation rate of 10⁻⁴ to 10⁻⁵ per nucleotide guarantees the preexistence of almost any mutation at any time point during HIV infection. Rapid evolution of the virus may facilitate the generation of variants that escape antiviral therapies.

As to the second factor, the intrinsic mutability of the viral target site in response to a specific antiviral agent can significantly affect the likelihood of resistant mutants. For example, zidovudine (AZT) selects for mutations in the reverse transcriptase of HIV more readily *in vitro* and *in vivo* than does stavudine.

With respect to the third factor, with increasing drug exposure, the selective pressure on the replicating virus population increases to promote the more rapid emergence of drug resistant mutants. For example, within a range of drugs that are efficacious for treatment of HIV, higher doses of AZT tend to select for drug resistant virus more rapidly than do lower doses (Richman et al. (1990) J. AIDS. 3, 743-6). This selective pressure for resistant mutants increases the likelihood of such mutants arising as long as significant levels of virus replication are sustained.

The fourth factor, the magnitude and rate of replication of the virus population, has major consequences on the likelihood of emergence of resistant mutants. Many virus infections are characterized by high levels of virus replication with high rates of virus turnover. This appears true of chronic infections with HIV. Higher levels of virus increase the probability of preexisting mutants. It has been shown that the emergence of a resistant population can result from the survival and selective proliferation of a previously existing subpopulation that randomly emerges in the absence of selective pressure. Therefore, a virus with a high level of replication in the presence of an added selective pressure such as an antiviral agent would be expected to produce larger numbers of resistant mutants. Evidence is accumulating that drug resistant mutants do in fact exist in subpopulations of HIV infected individuals (Najera et al. (1994) AIDS Res Hum Retroviruses 10, 1479-88; Najera et al. (1995) J Virol. 69, 23-31). The preexistence of drug resistant picornavirus mutants at a rate of approximately 10⁻⁵ is also well documented (Abroad et al. (1987) Antiviral Res. 8, 27-39). Below are classes of agents known to be administered to mammals at risk for or subject to HIV infection. Additional agents along with their HIV specific mutations are shown in Table II. For example, as discussed in more detail herein, the HIV-1 mutant polypeptide classified in Table II as mutational escape "V82A/F" has been observed to emerge as a result of administration of the agent Ritonavir, from the drug class of HIV protease inhibitors.

### Nucleoside reverse-transcriptase inhibitors

Nucleoside reverse-transcriptase inhibitors (NRTIs) were the first class of effective anti-retroviral compounds and zidovudine (AZT) was the first drug to reach clinical practice. Some of the NRTIs currently on the market include, zidovudine (Retrovir), stavudine (Zerit), didanosine (Videx), zalcitabine (Hivid), abacavir (Ziagen), lamivudine (Epivir), emtricitabine (Emtriva) and tenofovir (Viread). NRTIs inhibit the reverse transcriptase enzyme by competing with endogenous nucleosides for incorporation into the DNA chain generated by the reverse transcription of HIV RNA and thereby resulting in premature chain termination.

Researchers have isolated AZT-resistant reverse transcriptase and discovered sites that are frequently mutated in these enzymes. Met 41, Asp 67, and Lys70 from the "fingexs" domain and Leu 210, Thr 215, and Lys 219 from the "palm" domain are all mutations that are found near the active site in AZT-resistant reverse transcriptase. The manner in which these residues are clustered around the active site pocket tends to support that these mutations directly affect the binding site's ability to hold the azido group of the drug. Accordingly, provided herein are vectors, such as for example, yeast vehicles, that are associated with HIV mutant RT, including the HIV mutant polypeptides specifically described herein. In some examples, a yeast vehicle has been genetically engineered to comprise nucleic acid that encodes the HIV mutant RT polypeptide, or a fragment thereof that comprises the mutation.

Resistance to NRTIs develops from nucleoside changes within the reverse transcriptase (RT) gene and the subsequent generation of amino acid substitutions in the RT enzyme. Each NRTI induces a predictable set of genetic alterations, generally with primary mutations arriving first, and secondary mutations developing during continued therapy. For example, resistance to zidovudine is associated with the specific mutations in RT, including M41L, D67N, K70R, L210W, T215Y/F and K219Q/E. Mutations identified in RT associated with resistance to stavudine include but are not limited to M41L, K65R, D67N, K70R, Q151M, L210W, T215Y/F,and K219E. Mutations identified in RT associated with resistance to didanosine include but are not limited to K65R, L74V and M184V. Mutations identified in RT associated with resistance to zalcitabine include but are not limited to K65R/N, T69D, L74V, V75T/A and M184V. Mutations identified in RT associated with resistance to abacavir include but are not limited to, K65R, L74V, Y115F and M184V/I. Mutations identified in RT associated with resistance to lamivudine include but are not limited to, K65R and M184V/I. Mutations identified in RT associated with resistance to emtricitabine include but are not limited to K65R and M184V/I. Mutations identified in RT associated with resistance to tenofovir include but are not limited to, K65R. Most of these mutations are found near the active site of the reverse transcriptase enzyme.

The increasing use of sequential, alternating and combination nucleoside analogue regimens can select HIV variants with mutations that confer resistance to all the currently available NRTIs. Two sets of mutations have been described, the Q151M complex and the T69S insertion mutations. The Q151M mutations is often associated with secondary mutations at codons A62V, V75I, F77L and F116Y which further reduces the sensitivity of NRTIs.

### Non-nucleoside reverse transcriptase inhibitors

Non-nucleoside reverse transcriptase inhibitors (NNRTIs) are non-competitive inhibitors of HIV-1 reverse transcription and bind to a hydrophobic pocket near the active site of the reverse transcriptase, causing a conformational change in the enzyme. Contained in this hydrophobic pocket of reverse transcriptase are four residues that have been found to not mutate, namely Phe 227, Trp 229, and Leu 234 and Tyr 319. There are currently three approved NNRTIs, nevirapine (Viramune), delavirdine (Rescriptor) and efavirenz (Sustiva). Similar to NRTIs, HIV-1 variants with resistance to NNRTIs often have multiple mutations, however in contrast to most NRTIs, a single mutation can cause high levels of resistance to NNRTIs.

Nevirapine resistant HIV-1 variants arise very fast with decreased susceptibility evident after only 1 week of therapy and by 8 weeks of therapy 100% of patients tested had isolates with a greater than 100-fold decrease in susceptibility. Mutations identified in RT associated with resistance to nevirapine include but are not limited to, L100I, K103N, V106,A/M, V108I, Y181C/I, Y188C/L/H and G190A. Mutations identified in RT associated with resistance to delavirdine include but are not limited to, K103N, V106M, Y181C, Y18SL and P236L. Mutations identified in RT associated with resistance to efavirenz include but are not limited to, L100I, K103N, V106M, V108I, Y181C/I, Y188L, G190S/A and P225H. A rapid induction of resistance has been observed most strongly during NNRTI monotherapy and these compounds are approved to be taken in combination with other anti-retroviral agents.

### Protease inhibitors

The HIV protease enzyme is a dimeric aspartyl protease required for the post-translational cleavage of precursor Gag-Pol polyproteins during virus maturation, to generate building blocks required for assembly of new virus particles. The activity of this protein is essential for virus infectivity, rendering it a major drug target, Many protease inhibitors (PIs) are currently available and include indinavir (Crixivan), ritonavir (Norvir), saquinavir (Fortovase), nelfinavir (Viracept), amprenavir (Agenerase), lopinavir/ritonavir (Kaletra) and atazanavir (Reyataz). Mutations identified in RT associated with resistance to indinavir include but are not limited to, major mutations M46I/L, V82A/F/T and I84V, and other mutations L10I/R/V, K20M/R, L24I, V32I, M36I, I54V/A, L63P, I64V, A71T/V, G73S/A, V77I, and L90M. Mutations identified in RT associated with resistance to ritonavir include but are not limited to, major mutations V82A/F/T/S and I84V and other mutations L10F/I/R/V, K20M/R, V32I, L33F, M36I, M46I/L, I54V/L, A71V/T, V77I, and L90M. Mutations in isolates obtained from patients resistant to ritonavir appeared to occur in a stepwise and ordered fashion in sequence from V82A/F, I54V, A71V/T and I36L, followed by combinations of mutations at an additional 5 specific amino acid positions. Mutations identified in RT associated with resistance to saquinavir include but are not limited to, major mutations G48V and L90M and other mutations L10I/R/V, I54V/L, A71V/T, G73S, V77I, V82A, and I84V. Mutations identified in RT associated with resistance to nelfinavir include but are not limited to, major mutations D30N and L90M, and other mutations L10F/I, M36I, M46I/L, A71V/T, V77I, V82A/F/T/S, I84V and N88D/S. Mutations identified in RT associated with resistance to amprenavir include but are not limited to, major mutations V32I, M46I/L, I47V, I50V, I54L/M and 184V, and other mutations L10F/I/R/V, G73S and L90M. Mutations identified in RT associated with resistance to atazanavir include but are not limited to, major mutations M46I, I50L, A71V, I84V and N88S, and other mutations L10I/F/V, K20R/M/I, L24I, V32I, L33I/F, M36I/L/V, G48V, I54L, G73C/S/T/A, V82A and L90M. Accordingly, provided herein are vectors, such as yeast vehicles that are associated with HIV mutant protease, including the HIV mutant polypeptides specifically described herein. In some examples, a yeast vehicle has been genetically engineered to comprise nucleic acid that encodes the HIV mutant protease polypeptide, or fragment thereof that comprises the mutation.

### Entry/fusion inhibitors

Enfuvirtide (Fuzeon) was the first in a class of antiviral agents designed to inhibit viral entry. Unlike existing anti-HIV drugs, which target viral enzymes involved in replication of the virus, enfuvirtide was designed to block HIV from fusing with a host cell. Enfuvirtide competitively binds to a motif (HR1) within the HIV gp41 ectodomain and blocks formation of a structure that is a prerequisite for membrane fusion and viral entry. Mutations associated with reduced susceptibility have been observed and include but are not limited to G36D/S, I37V, V38A/M, Q39R, N42T and N43D. Specifically, the change of valine to alanine at amino acid 38 was shown to provide a 45-fold resistance to enfuvirtide. Accordingly, provided herein are vectors, such as yeast vehicles, that are associated with HIV mutations G36D/S, I37V, V38A/M, Q39R, N42T and N43D. One of skill in the art will appreciate that additional HIV mutant polypeptides may emerge as new agents for prophylactic and therapeutic administration are developed. Such HIV mutant polypeptides may be identified by methods deemed routine to the skilled artisan and used in compositions and methods as described herein. Accordingly, provided herein are compositions comprising a mutant polypeptide of HIV (or mimetope thereof) or nucleic acid encoding a mutant polypeptide of HIV and methods for their use in eliciting an immune response. In some examples, the immune response is a cellular immune response. In some examples, the immune response is an humoral response. In other examples, the immune response includes both cellular and humoral responses.

### Hepatitis C Virus

Hepatitis C virus (HCV) is a single-stranded virus with a 9.6-kb genome, which encodes a polyprotein precursor of about 3000 amino acids. All of the protein products of HCV are produced by proteolytic cleavage of this large polyprotein. The proteolytic cleavage is carried out by one of three proteases: the host signal peptidase, the viral self-cleaving metalloproteinase, NS2, or the viral serine protease NS3/4A. The combined action of these enzymes produces the structural proteins (C, E1 and E2) and non-structural (NS2, NS3, NS4A, NS4B, NS5A, and NS5B) proteins which are required for replication and packaging of viral genomic RNA. NS5B is the viral RNA-dependent RNA polymerase (RDRP) that is responsible for the conversion of the input genomic RNA into a negative stranded copy (complimentary RNA, or cRNA); the cRNA then serves as a template for transcription by NS5B of more positive sense genomic/messenger RNA.

The NS3 serine protease and the RNA polymerase have been major targets for the development of HCV-specific therapeutics over the last decade. Interferon and ribavirin are two drugs currently licensed for the treatment of individuals with chronic hepatitis C infection. Interferon is administered alone or in combination with ribavirin. This combination therapy has demonstrated a sustained antiviral effect in only 40-50% of genotype 1 HCV-infected patients and in 80-90% of patients infected with HCV genotypes 2 or 3.

HCV serine protease inhibitors BILN 2061 and VX-950 have entered clinical trials. It was reported that these protease inhibitors were tested *in vitro* to determine whether resistant mutants would emerge during treatment and to characterize the mutants that did arise. Distinct drug-resistant substitutions of a single amino acid were identified in the HCV NS3 serine protease domain for both inhibitors. The major BILN 2061-resistance mutation was at amino acid 168 consisting of a Val substitution (D 168V) 64% of the time, or an Ala substitution (D168A) 24% of the time. In contrast, the major VX-950-resistance mutation was at amino acid 156 consisting of a Ser substitution (A156S) in 79% of the samples. (Lin C. etal. JBC, (2004) 279:17508-17514. See Table III for mutations associated with agents for use in treating mammals subject to HCV and HBV.

**TABLE III**

| | | | |
|---|---|---|---|
| Virology/Indication | Therapeutic Agent | Target | Mutational Escape |
| HCV | VX-950 | HCV Protease | A156S |
| HCV | BILN 2061 | HCV Protease | D168V |
| | | | D168A |
| HBV | Lamivudine | HBV Polymerase | M552V/I |
| | | | L528M |
| HBV | Hepsera | HBV Polymerase | rtN236T (using rt nomenclature) |
| | | | *rt*A181V |
| HBV | Entecavir | HBV Polymerase | rtM250V |
| | | | rtT184G |
| | | | rtS202I |
| | | | rtI169T |

A mutation can be located at any position of the HCV polypeptide. Although the mutations are described in the context of single mutations, it is to be understood that the viral antigen can comprise more than one (such as two, three, four, or five) mutations that are associated with the loss of susceptibility to an anti-viral agent.

Accordingly, provided herein are compositions comprising a mutant polypeptide of HCV, a fragment thereof that comprise a mutation, or a mimetope, or nucleic acid encoding a mutant polypeptide of HCV and methods for their use in eliciting an immune response. In some examples, the immune response is a cellular immune response. In some examples, the immune response is an humoral response. In other examples, the immune response includes both cellular and humoral responses. In some examples, a mutant polypeptide comprises a mutation in NS3 protease protein from hepatitis C virus, including but not limited to, the mutants shown in Table III.

### Hepatitis B Virus

Hepatitis B virus (HBV) infection is a global health problem. It is estimated that more than 2 billion people are infected with HBV (as of 2004) and more than 350 million are chronic carriers. HBV infection acquired in adult life is often clinically inapparent, and most acutely infected adults recover completely from the disease and clear the virus. Rarely, however, the acute liver disease may be so severe that the patient dies of fulminant hepatitis. A small fraction, perhaps 5 to 10%, of acutely infected adults, becomes persistently infected by the virus. However, according to the Centers for Disease Control and Prevention up to 90% of infants infected at birth and 30% of children infected at age 1-5 years become chronically infected. HBV chronically infected individuals develop liver diseases of varying severity with 15-25% dying from cirrhosis and primary hepatocellular carcinoma.

HCV is a member of the Hepadnaviridae family of viruses which also includes the duck hepatitis B virus and the woodchuck hepatitis B virus. HBV has a 3.2kb genome with four partially overlapping open reading frames encoding structural envelope or surface proteins, (HBsAg), precore/core proteins (HBeAg and HBcAg), the reverse transcription-DNA polymerase enzyme and a transactivator X protein. Partly double-stranded genomic DNA is encapsidated by an assembly of core protein dimers. This nucleocapsid in turn is surrounded by an envelope that consists of a host cell-derived lipid bilayer and viral surface proteins.

The viral life cycle of HBV includes an intracellular pre-genomic RNA that is reverse transcribed to DNA within the assembled viral nucleocapsid. Because the reverse transcriptase-DNA polymerase lacks proof-reading ability, HBV exhibits a mutation rate that is more than 10-fold higher than other DNA viruses; the estimated mutation rate is approximately 1 nucleotide/10,000 bases/infection year. This high mutation rate and genome variability is reflected in the occurrence of serologically defined subtypes and by six different genotypic groups characterized to date, designated A through F.

Currently two major categories of drugs are used to treat HBV infection:
nucleoside analogues and immunomodulators. The immunomodulators include interferon-α,
thymosin-α and therapeutic vaccines, although only interferon-α is currently approved for use in patients. Immunomodulators are believed to promote destruction of infected liver cells and to stimulate cytokine production to suppress HBV replication. In contrast, nucleoside analogues interfere with HBV-DNA synthesis and are believed to accelerate clearance of infected cells. The most common nucleoside analogue drugs used to treat HBV infection are lamivudine and adefovir dipivoxil. In addition, entecavir was recently approved for treatment of chronic HBV infection.

Nucleoside analogues such as lamivudine, adefovir and entecavir are chemically synthesized compounds which are structurally similar to natural nucleosides. As such, they are incorporated into newly synthesized HBV-DNA causing premature chain termination and resulting in inhibition of viral replication. In addition, some nucleoside analogues appear to competitively inhibit the DNA-dependent and reverse transcriptase activity of the viral polymerase which also results in inhibition of viral replication.

Lamivudine, also known as 3TC and Epivir, is a cytidine analogue which acts by terminating viral DNA synthesis and competitively inhibiting viral polymerase/reverse transcriptase. Of major concern to practitioners is the emergence of drug-resistant HBV variants following lamivudine treatment. Drug resistance has been demonstrated in up to 66% of all patients after prolonged periods of treatment.

The HBV polymerase, is divided into four domains, one of which functions as the reverse transcriptase of the virus and contains at least five subdomains (A-E). Similar to other RNA-dependent polymerases, the HBV polymerase contains a characteristic YMDD (tyrosine-methionine-aspartate-aspartate) motif at the catalytic site, which is located within subdomain C. There are now two numbering systems for the HBV polymerase, the original system numbered amino acids of the entire polymerase while the new system numbers amino acids of each polymerase domain separately, thereby the amino acids of the reverse transcriptase domain are designed with an "rt".

The most common amino acid substitutions that have been described to confer lamivudine resistance occur in both the B and C subdomains. Amino acid substitutions that are associated with drug resistance predominantly affect the YMDD motif so that the methionine (M) at position rt244 (new rt numbering system, 552 old system) is changed either to valine or isoleucine, rtM204V/I (M552V/I). This mutation is almost always associated with a second mutation in subdomain B, a substitution of the leucine at position *rt1*80 with a methionine, rtL180M (L528M). Other mutations that have been described include the rtV173L (V521L) and rtF166L (F541L) both in subdomain B. Another mutant, rtA181T (A529T) has been shown to be resistant to lamivudine following prolonged treatment and more recently, a *rt*M204S (M552S) mutant has been described, usually associated with a *rt*L180M (L528M) change.

Adefovir dipivoxil (Hepsera) is an acyclic nucleotide analog of adenosine monophosphate which has been shown to be a potent inhibitor of HBV replication. As with lamivudine, genotypic changes conferring reduced susceptibility to adefovir have been observed. It was reported that analyses of viral mutants isolated at 96-144 weeks post treatment determined that mutations *rt*N236T and *rt*A181V contribute to adefovir resistance. Entecavir (Baraclude) is a guanosine nucleoside analogue which has also shown activity against HBV polymerase. In patients refractory to lamivudine treatment, after treatment with entecavir for 48 weeks, there was evidence of emerging resistance associated with amino acid substitutions including but not limited to *rt*I169T, *rt*T184G, *rt*S202I and *rt*M250V when preexisting lamivudine resistance mutations *rt*L180M and/or *rt*m204V/I were present.

There are a number of other nucleoside analogues that have been tested or are presently being evaluated against HBV in clinical studies. These include famciclovir, emtricitabine and ganciclovir. It has been reported that, similar to laminvudine usage, prolonged treatment with famciclovir results in drug resistance. To date a number of mutations have been described effecting amino acids in the B and C subdomains of the reverse trnscriptase including, but not limited to, *rt*L180M, *rt*V173L, *rt*P177L, *rt*T184S, *rt*R153A and *rt*V207I.

Accordingly, provided herein are compositions comprising a mutant polypeptide of HBV, a fragment thereof that comprises a mutation, or mimetope thereof or nucleic acid encoding a mutant polypeptide of HBV and methods for their use in eliciting an immune response. In some examples, the immune response is a cellular immune response. In some examples, the immune response is an humoral response. In other examples, the immune response includes both cellular and humoral responses. In some examples, a mutant polypeptide of HBV comprises a mutation shown in Table III.

Accordingly, the compositions described herein are used for eliciting an immune response to a mutant polypeptide in a mammal, comprising administration to the mammal of an effective amount of the composition in conjunction with the targeted therapeutic and/or prophylactic agent. Further the compositions may be used in preparation of or manufacture of medicaments for eliciting an immune response to a mutant polypeptide in a mammal in conjunction with a targeted therapeutic and/or prophylactic agent. In some examples, the mutant polypeptide is a polypeptide encoded by a virus. In some examples the mutant polypeptide is encoded by a virus is selected from the group consisting of retrovirus, flavivirus, reovirus, picornavirus, coronavirus, filovirus, rhabdovirus, bunyaviurs, orthomyxovirus, paramyxovirus, arenavirus, calicivirus, hepadnavirus, herpesvirus, poxvirus, adenovirus, parvovirus and papovavirus. In other examples the virus is HIV, HBV or HCV.

In some examples the immune response is a cellular immune response, In other examples the immune response is an humoral immune response. In yet other examples the immune response includes both a cellular and humoral immune response.

In addition, the compositions described herein are used to treat a disease in a mammal, comprising administration to the mammal of an effective amount of the composition, wherein the disease is associated with a mutant polypeptide that is known to emerge or has emerged with at least one specific mutation in response to administration of a targeted therapeutic and/or prophylactic agent. In some examples the compositions are used in conjunction with the targeted therapeutic and/or prophylactic agent. Further, the compositions may be used in manufacture of medicaments for treating the disease in a mammal in conjunction with a targeted therapeutic and/or prophylactic agent. In some examples, the mutant polypeptide is a polypeptide encoded by a virus. In some examples the virus is selected from the group consisting of retrovirus, flavivirus, reovirus, picornavirus, coronavirus, filovirus, rhabdovirus, bunyaviurs, orthomyxovirus, paramyxovirus, arenavirus, calicivirus, hepadnavirus, herpesvirus, poxvirus, adenovirus, parvovirus and papovavirus. In other examples the virus is HIV, HBV or HBV. In some examples the disease is infection with HIV, HBV or HCV.

### Fusion protein

In some examples, provided herein are vectors and viruses comprising a fusion protein that comprises at least one mutant polypeptide that is known to emerge or has emerged in response to an agent. In some examples, the fusion protein comprises additional mutant polypeptides and in other examples comprises an adjuvant, such as a ligand for a TLR. In some examples, provided herein are vectors, including yeast vehicles, comprising such fusion proteins. A fusion protein can contain any mutant polypeptide as described herein or known in the art. By way of illustration, one such fusion construct provided herein is a fusion protein that comprises (a) at least one HIV mutant polypeptide or antigen, such as a mutant RT or protease (including immunogenic domains and epitopes of a full-length antigen, as well as various fusion proteins and multiple antigen constructs as described elsewhere herein, as long as the antigen contains the desired mutation); and (b) a synthetic polypeptide(s), antigen(s), or peptide(s). The fusion protein may contain multiple immunogenic domains, from one or more HIV mutant polypeptides. Such fusion proteins are particularly useful when it is desirable to encompass several different HIV mutant polypeptides and/or combinations of HIV mutant polypeptides that may occur at one or a few positions in the antigen in nature, in a single vector, such as a yeast vehicle. In some examples, a yeast vehicle comprises a fusion protein comprising two or more HIV mutant polypeptides or antigens. In some examples, the fusion protein comprises two or more epitopes of one or more HIV mutant antigens, or immunogenic domains, and in some examples, multiple domains, of an immunogenic antigen, wherein the multiple domains together encompass several different mutations and/or combinations of mutations that may occur at one or more amino acid positions in the HIV mutant antigen in response to an agent.

For example, as shown in Table II, there are several mutations identified in HIV RT associated with resistance to several drugs, such as for example, L74V, K65R and M184V/I. The present invention encompasses vectors and viruses, including yeast vectors and vehicles, and compositions comprising them, that are in association with or express fusion proteins comprising multiple HIV mutant polypeptides, such as for example, at least one of L74V, K65R and M184V/I, that are known to emerge in response to RT inhibitors. As shown in Table II, there are several mutations identified in the HIV protease, such as for example, V82A/F, I54V, A71V/T, I36L, I50L, N88S, 184V, A71V, M46I, G48V, L90M, L101RN/VI54V/L, A71VT, G73S, V77I, V82A, and I84V. The present invention encompasses vectors and viruses, including yeast vectors and vehicles, and compositions comprising them, that are in association with or express fusion proteins comprising multiple HIV mutant polypeptides, such as for example, at least one of V82A/F, I54V, A71V/T, I36L, I50L, N88S, 184V, A71V, M46I, G48V, L90M, L101R/VI54V/L, A71VT, G73S, V77I, V82A, and 184V, that emerge in response to protease inhibitors.

### Compositions and Methods of Use

Provided herein are compositions and methods for their use in eliciting an immune response, in some examples, a cellular immune response, an humoral immune response, or a cellular and humeral immune response, to a mutant polypeptide that is known to emerge or that has emerged in response to a targeted drug agent, or to a cell that expresses the mutant polypeptide. In some examples, the cellular immune response is intended to eliminate the cell, such as for example, a cancer cell or cell that expresses a virus, that has escaped control and comprises mutations in the polypeptide targeted by the drug agent, but this is not required. In some examples, the cellular or humoral immune response will block cell proliferation or viral replication.

Provided herein are vectors, viruses and compositions, such as immunogenic compositions, that comprise a mutant polypeptide, a fragment thereof that comprises a mutation, a mimetope (or nucleic acids encoding thereof) that is known to emerge or has emerged with a specific mutation in response to a therapeutic and/or prophylactic agent(s), and methods for their use in eliciting an immune response, in some examples, a cellular immune response, to the mutant polypeptide. A humoral immune response may also be elicited. Emergence of such mutant polypeptides in response to such agent(s) is believed to be associated with, for example, decreased susceptibility and/or resistance to the agent(s) and/or an increase in sub-clinical and/or clinical symptoms of disease. As described in Stubbs et al.(Nature Medicine, 200 1, vol.7: 1-5), a yeast-based composition, that is, whole recombinant *S. cerevisiae* yeast expressing HIV-1-_{sf2}-gp160 envelope protein administered to mice, as described therein, elicited a CTL immune response. CTL generated from the mice were able to kill target cells expression gp160-SF2. The benefit of eliciting a cellular immune response and/or humoral immune response to a HIV mutant polypeptide that is known to emerge or has emerged with specific mutations in response to an agent that will be, has been or is being administered to a mammal in need may include extending the duration of the agent effectiveness; minimizing or reversing resistance to the agent; delaying or minimizing emergence of the specific HIV mutant polypeptide; eliminating the specific HIV mutant polypeptide (although elimination is not required in order to have a benefit); and minimizing, reducing or reversing a symptom of HIV infection, and/or slowing progression of HIV infection.

Accordingly, provided herein are methods for eliciting an immune response to a mutant polypeptide, including an HIV mutant polypeptide, that is known to emerge or has emerged with a specific mutation in response to a therapeutic and/or prophylactic agent(s) in a mammal that comprises administering to the mammal an elective amount of a composition in conjunction with the agent, wherein the composition comprises,
a. a cell, vector or virus comprising nucleic acid that encodes the mutant polypeptide;
b. a cell, vector or virus in association with the mutant polypeptide;
c. the mutant polypeptide, or a peptide (mimetope) that elicits an immune response to the mutant polypeptide; or
d. nucleic acid encoding the mutant polypeptide, or nucleic acid, such as siRNA or anti-sense RNA that binds the nucleic acid,
wherein an effective amount of the composition is administered in conjunction with the agent.

In some examples, the composition comprises one or more of the following:
i) a yeast vehicle comprising nucleic acid which encodes at least one mutant polypeptide, a fragment thereof that comprises a mutation, or a mimetope;
ii) a yeast vehicle comprising at least one mutant polypeptide, a fragment thereof that comprises a mutation, or a mimetope;
iii) a yeast vehicle in association with at least one mutant polypeptide, a fragment thereof that comprises a mutation, or a mimetope;
iv) a yeast vehicle comprising nucleic acid which encodes at least one mutant polypeptide, a fragment thereof that comprises a mutation, or a mimetope loaded intracellularly into a dendritic cell; or
v) a yeast vehicle and at least one mutant polypeptide, a fragment thereof that comprises a mutation, or a mimetope, loaded intracellularly into a dendritic cell,
wherein the mutant polypeptide is known to emerge or has emerged with at least one specific mutation in response to administration of a targeted therapeutic and/or prophylactic agent.

In some examples, the composition is capable of eliciting a cellular immune response. In other examples, the immune response is a humoral immune response. In other examples, the immune response includes both a cellular and humeral immune response.

In some examples of the methods, the composition comprises an adjuvant. In yet other examples, the composition further comprises an agonist or ligand for a Toll-like receptor. In other examples, the composition comprises a yeast vehicle. In yet other examples, the composition comprises a CpG sequence. In other examples, the cell is a dendritic cell. In some examples, the mammal is a human.

In some examples, wherein the agent is known to interfere with cell proliferation, either directly or indirectly, administration of a mutant polypeptide, or nucleic acid encoding the mutant polypeptide, includes administration prior to and/or after administration of the agent, but not concurrent with administration of the agent. In some examples, a HIV mutant polypeptide is L74V, K65R and/or M184V/I, that are known to emerge in response to HIV RT inhibitors. In other examples, the HIV mutant polypeptide is V82A/F, I54V, A71V/T, I36L, I50L, N88S, I84V, A71V, M46I, G48V, L90M, L101R/VI54V/L, A71VT, G73S, V77I, V82A, and/or 184V, that are known to emerge in response to HIV protease inhibitors. In other examples, a mutant polypeptide of a cancer antigen is selected from the group shown in Table I. In other examples, a mutant polypeptide of HCV or HBV is selected from the group shown in Table HI.

Provided herein are methods for ameliorating a symptom of disease in a mammal, comprising administering to the mammal an effective amount of a composition in conjunction with a therapeutic and/or prophylactic agent, wherein the composition comprises,
a. a cell, vector or virus comprising nucleic acid that encodes the mutant polypeptide;
b. a cell, vector or virus in association with the mutant polypeptide;
c. the mutant polypeptide, or a peptide (mimetope) that elicits an immune response to the mutant polypeptide; or
d. nucleic acid encoding the mutant polypeptide, or nucleic acid, such as siRNA or anti-sense RNA that binds the nucleic acid,
wherein an effective amount of the composition is administered in conjunction with the agent.

In some examples, the composition comprises one or more of the following:
i) a yeast vehicle comprising nucleic acid which encodes at least one mutant polypeptide, a fragment thereof that comprises a mutation, or a mimetope;
ii) a yeast vehicle comprising at least one mutant polypeptide, a fragment thereof that comprises a mutation, or a mimetope;
iii) a yeast vehicle in association with at least one mutant polypeptide, a fragment thereof that comprises a mutation, or a mimetope;
iv) a yeast vehicle comprising nucleic acid which encodes at least one mutant polypeptide, a fragment thereof that comprises a mutation, or a mimetope loaded intracellularly into a dendritic cell; or
v) a yeast vehicle and at least one mutant polypeptide, a fragment thereof that comprises a mutation, or a mimetope loaded intracellularly into a dendritic cell,
wherein the mutant polypeptide is known to emerge or has emerged with at least one specific mutation in response to administration of a targeted therapeutic and/or prophylactic agent.

In some examples, the composition is capable of eliciting a cellular immune response. In some examples, the immune response is a humoral immune response. In other examples, the immune response includes both a cellular response and a humoral response.

In some examples of the methods, the composition comprises an adjuvant. In other examples, the composition comprises a yeast vehicle. In other examples, the composition comprises a CpG sequence. In yet other examples, the composition further comprises a ligand for a Toll-like receptor. In other examples, the cell is a dendritic cell.

In some examples, the mammal is a human.

In some examples of the methods, the mammal is at risk for disease and is being administered the agent prophylactically; in other examples, the mammal is subject to the disease and the agent is being administered the agent therapeutically. U.S. Patent No. 5,830,463, related to yeast systems, demonstrates that yeast engineered to express a heterologous antigen is capable of eliciting both cellular and humoral immune responses when administered to a mammal. Accordingly, provided herein are yeast vehicles, including yeast vectors; yeast-based compositions; and methods for their use in eliciting an immune response to a mutant polypeptide that is known to emerge or has emerged with a specific mutation in response to a therapeutic and/or prophylactic agent(s). In some examples, the yeast vehicle comprises nucleic acid encoding the mutant polypeptide(s). In other examples, the yeast vehicle is in association with the mutant polypeptide. In some examples, the composition comprises one or more of the following:
i) a yeast vehicle comprising nucleic acid which encodes at least one mutant polypeptide, a fragment thereof that comprises a mutation, or a mimetope;
ii) a yeast vehicle comprising at least one mutant polypeptide, a fragment thereof that comprises a mutation, or a mimetope;
iii) a yeast vehicle in association with at least one mutant polypeptide, a fragment thereof that comprises a mutation or a mimetope;
iv) a yeast vehicle comprising nucleic acid which encodes at least one mutant polypeptide, a fragment thereof that comprises a mutation, or a mimetope loaded intracellularly into a dendritic cell; or
v) a yeast vehicle and at least one mutant polypeptide, a fragment thereof that comprises a mutation, or a mimetope loaded intracellularly into a dendritic cell,
wherein the mutant polypeptide is known to emerge or has emerged with at least one specific mutation in response to administration of a targeted therapeutic and/or prophylactic agent. Accordingly provided herein are yeast vehicles and yeast-based compositions for use in the methods as described herein.

A mutant polypeptide that is known to emerge or has emerged in response to an agent, or nucleic acid encoding the polypeptide, may be administered with or without an adjuvant, as long as the polypeptide or nucleic acid encoding the polypeptide is capable of eliciting a cellular immune response, which can be measured by methods known in the art, and includes for example, measuring the presence cytokines and/or chemokines, such as for example, MIP-1a, MIP-1b and/or RANTES. In the case of HIV, for example, assays of antigen-specific T cell responses directed against the specific HIV mutant polypeptide, including lymphocyte proliferation assays for monitoring CD4 responses and CTL assays, can be used to monitor efficacy of the compositions and methods disclosed herein. Alternatively, efficacy can be assayed by measuring immune response against challenge with tumors expressing the HIV mutant polypeptide. A HIV mutant polypeptide that is not capable of eliciting a cellular immune response on its own without an adjuvant, can be administered with an adjuvant, as described herein.

Provided herein are compositions comprising vectors in association with a mutant polypeptide, including compositions to be administered to a patient directly or first loaded into a carrier such as a dendritic cell, using a number of techniques known to those skilled in the art. For example, vectors can be dried by lyophilization or frozen by exposure to liquid nitrogen or dry ice. Compositions comprising yeast vehicles can also be prepared by packing yeast in a cake or a tablet, such as is done for yeast used in baking or brewing operations. In addition, prior to loading into a dendritic cell, or other type of administration, vectors can also be mixed with a pharmaceutically acceptable excipient, such as an isotonic buffer that is tolerated by the host cell. Examples of such excipients include water, saline, Ringer's solution, dextrose solution, Hank's solution, and other aqueous physiologically balanced salt solutions. Nonaqueous vehicles, such as fixed oils, sesame oil, ethyl oleate, or triglycerides may also be used. Other useful formulations include suspensions containing viscosity enhancing agents, such as polyethylene glycols (PEG) sodium carboxymethylcellulose, sorbitol, glycerol or dextran. Excipients can also contain minor amounts of additives, such as substances that enhance isotonicity and chemical stability. Examples of buffers include phosphate buffer, bicarbonate buffer and Tris buffer, while examples of preservatives include thimerosal, m- or o-cresol, formalin and benzyl alcohol. Standard formulations can either be liquid injectables or solids which can be taken up in a suitable liquid as a suspension or solution for injection. Thus, in a non-liquid formulation, the excipient can comprise, for example, dextrose, human serum albumin, and/or preservatives to which sterile water or saline can be added prior to administration.

Provided herein are methods comprising administering a composition (such as an immunogenic composition) that comprises a vector in association with a mutant antigen to a mammal at risk for cancer or infection or subject to cancer or infection. The methods are generally useful for eliciting an immune response, which in some examples, is an cellular immune response in the mammal. Such methods are believed to be useful in eliciting a cellular immune response to a mutant polypeptide that has emerged in response to an agent(s) or is believed will emerge in response to an agent; thereby minimizing or reversing resistance to the agent, and/or extending the efficacy of the agent and/or minimizing, reducing or reversing some symptoms of disease or infection.

Accordingly, provided herein are methods for minimizing resistance to a prophylactic and/or therapeutic agent in a mammal, comprising administering to the mammal a effective amount of a composition comprising a vector, such as for example, a yeast vehicle, in association with a mutant polypeptide that has emerged in response to the agent. Also, provided herein are methods for reducing resistance to an agent administered to a mammal at risk of disease or infection or subject to disease or infection, whether the agent is administered prophylactically and/or therapeutically, comprising administering to the mammal an effective amount of a composition in conjunction with the agent, wherein said composition comprises,
a. a cell, vector or virus comprising nucleic acid that encodes the mutant polypeptide;
b. a cell, vector or virus in association with the mutant polypeptide;
c. the mutant polypeptide, or a peptide (mimetope) that elicits an immune response to the mutant polypeptide; or
d. nucleic acid encoding the mutant polypeptide, or nucleic acid, such as siRNA or anti-sense RNA that binds the nucleic acid,
wherein an effective amount of the composition is administered in conjunction with the agent.

In some examples, the composition comprises one or more of the following:
i) a yeast vehicle comprising nucleic acid which encodes at least one mutant polypeptide, a fragment thereof that comprises a mutation, or a mimetope;
ii) a yeast vehicle comprising at least one mutant polypeptide, a fragment thereof that comprises a mutation, or a mimetope;
iii) a yeast vehicle in association with at least one mutant polypeptide, a fragment thereof that comprises a mutation, or a mimetope;
iv) a yeast vehicle comprising nucleic acid which encodes at least one mutant polypeptide, a fragment thereof that comprises a mutation, or a mimetope loaded intracellularly into a dendritic cell; or
v) a yeast vehicle and at least one mutant polypeptide, a fragment thereof that comprises a mutation, or a mimetope loaded intracellularly into a dendritic cell,
wherein the mutant polypeptide is known to emerge or has emerged with at least one specific mutation in response to administration of a targeted therapeutic and/or prophylactic agent.

In some examples, the composition is capable of eliciting a cellular immune response. In other examples, the immune response is a humoral immune response. In other examples, the immune response includes both a cellular and humeral immune response.

In some examples of the methods, the composition comprises an adjuvant. In yet other examples, the composition further comprises an agonist or ligand for a Toll-like receptor. In other examples, the composition comprises a yeast vehicle. In yet other examples, the composition comprises a CpG sequence. In other examples, the cell is a dendritic cell. In some examples, the mammal is a human.

In further examples, the mutant polypeptide is a HIV mutant polypeptide comprising L74V, K65R and/or M184V/I, that are known to emerge in response to HIV RT inhibitors. In other examples, the HIV mutant polypeptide is V82A/F, I54V, A71V/T, I36L, 150L, N88S, I84V, A71V, M46I, G48V, L90M, L101R/VI54V/L, A71VT, G73S, V77I, V82A, and/or 184V, that are known to emerge in response to HIV protease inhibitors. Examples of agents include, for example, HIV RT inhibitors, such as for example, abacavir, tenofovir, didanosine and lamivudine, emtricitabine, stavudine and zalcitabine. Examples of agents include for example, HIV protease inhibitors such as for example, saquinavir, nelfinavir and ritonavir, indinavir and atazanavir. Examples of other mutant polypeptides and agents are described herein in Tables I and III.

Also provided herein are vectors, including for example, yeast vehicles, viruses and compositions, such as, for example, yeast-based compositions comprising yeast vehicles, including immunogenic compositions, for use in methods for eliciting a mutant polypeptide specific immune response in a mammal that has been, will be, or is being administered the agent(s). In some examples, the mammal is at risk for a disease, and a vector associated with a mutant polypeptide, a fragment thereof that comprises a mutation or a mimetope, and/or compositions comprising such vector, is administered prophylactically, before, concurrently with and/or after the agent. In other examples, the mammal is subject to disease and a vector associated with a mutant polypeptide, and/or compositions comprising such vectors, is administered therapeutically, before, concurrently with and/or after the agent. Administration of such yeast vehicles in association with a mutant polypeptide may be used, for example, to increase susceptibility of the mammal to a therapeutic and/or prophylactic agent; and/or to increase therapeutic efficacy of such agents; and/or to extend the effective life cycle of such agents. In some examples, a mutant polypeptide is identified prior to administration of a vector or composition as described herein, and in other examples, a mutant polypeptide is predicted to occur in response to an agent.

Compositions described herein that comprise vectors, such as yeast vehicles, in association with a mutant polypeptide, a fragment thereof that comprises a mutation or a mimetope, and the therapeutic or prophylactic agent can be administered either simultaneously or sequentially, whether prior to or after administration of the agent(s). Simultaneous administration encompasses administration together in one composition or alternatively, as separate compositions. In some examples, the agent and mutant polypeptide, or nucleic acid encoding it, are in different formulations and are administered simultaneously and separately. As will be appreciated, in some examples, wherein the agent and mutant polypeptide, or nucleic acid encoding it, are administered sequentially, the administration may be on a daily, weekly, or monthly basis as will be deemed appropriate by the practitioner for the mammal. The term "simultaneous administration" as used herein, means that the composition comprising the yeast vehicle and the therapeutic agent are administered on the same day. Either the composition comprising the mutant polypeptide or the therapeutic agent may be administered first. When administered simultaneously, the composition comprising the yeast vehicle and the therapeutic agent may be contained in the same dosage (i.e., a unit dosage comprising both the composition comprising the yeast vehicle and the therapeutic agent) or in discrete dosages (e.g., the composition comprising the yeast vehicle is contained in one dosage form and the therapeutic agent is contained in another dosage form).

In some examples, the composition comprising the mutant polypeptide is administered as a "follow-up treatment," i.e., after treatment of the agent has been initiated or after an increase in a symptom of disease is observed. However, the composition comprising the vector, such as a yeast vehicle, can also be administered before treatment with the therapeutic or prophylactic agent has been initiated.

The methods described herein may also comprise administering a vector and a mutant polypeptide to a mammal, wherein the vector and the polypeptide are not complexed with each other, i.e., the polypeptide is not recombinantly expressed by the vector, loaded into the vector, or physically attached to the vector. The vector and the mutant polypeptide can be mixed in a formulation prior to administration to the subject, or administered separately. The administration process can be performed *ex vivo,* such as by introduction by dendritic cells loaded with the yeast vehicle, or *in vivo. Ex vivo* administration refers to performing part of the regulatory step outside of the mammal, such as administering a composition of the present invention to a population of cells (dendritic cells) removed from a mammal under conditions such that the vector and mutant polypeptide are loaded into the cell, and returning the cells to the mammal. The composition comprising a vector can then be returned to a mammal, or administered to a mammal, by any suitable mode of administration.

Administration of a composition, including a composition comprising a dendritic cell loaded with a vector and mutant polypeptide, can be, for example, systemic, or mucosal. The routes of administration will be apparent to those of skill in the art, depending on the type of condition, the mutant polypeptide used, and/or the target cell population or tissue. Methods of administration include, but are not limited to, intravenous administration, intraperitoneal administration, intramuscular administration, intranodal administration, intracoronary administration, intraarterial administration (e.g., into a carotid artery), subcutaneous administration, transdermal delivery, intratracheal administration, subcutaneous administration, intraarticular administration, intraventricular administration, inhalation (e.g., aerosol), intracranial, intraspinal, intraocular, aural, intranasal, oral, pulmonary administration, impregnation of a catheter, and direct injection into a tissue. Routes of administration include: intravenous, intraperitoneal, subcutaneous, intradermal, intranodal, intramuscular, transdermal, inhaled, intranasal, oral, intraocular, intraarticular, intracranial, and intraspinal. Parenteral delivery can include intradermal, intramuscular, intraperitoneal, intrapleural, intrapulmonary, intravenous, subcutaneous, atrial catheter and venal catheter routes. Aural delivery can include ear drops, intranasal delivery can include nose drops or intranasal injection, and intraocular delivery can include eye drops. Aerosol (inhalation) delivery can also be performed using methods standard in the art (see, for example, Stribling et al., Proc. Natl. Acad. Sci. USA 189:11277-11281, 1992, which is incorporated herein by reference in its entirety). For example, in one example, a composition comprising a yeast vehicle can be formulated into a composition suitable for nebulized delivery using a suitable inhalation device or nebulizer. Oral delivery can include solids and liquids that can be taken through the mouth, and is useful in the development of mucosal immunity and since compositions comprising yeast vehicles can be easily prepared for oral delivery, for example, as tablets or capsules, as well as being formulated into food and beverage products. Other routes of administration that modulate mucosal immunity are useful in the treatment of cancer or infectious disease. Such routes include bronchial, intradermal, intramuscular, intranasal, other inhalatory, rectal, subcutaneous, topical, transdermal, vaginal and urethral routes. The route of delivery is any route of delivery of a composition comprising a yeast vehicle to the respiratory system, including, but not limited to, inhalation, intranasal, intratracheal, and the like.

Effective administration of a cell, vector, such as a yeast vehicle, or virus, or composition comprising a cell, vector, virus or yeast vehicle as described herein to a mammal at risk for or subject to disease does not require that the mammal is protected from the disease. Effective dose parameters can be determined using methods standard in the art that are suitable to minimize or reduce symptoms of disease, or minimize progression of the disease. Such methods include, for example, determination of survival rates, side effects (i.e., toxicity) and progression or regression of disease.

For use with a yeast vehicle, a suitable single dose size is a dose that is capable of eliciting an immune response in a mammal, in some examples, a cellular immune response, which may be an antigen-specific immune response, when administered one or more times over a suitable time period. As will be understood by the skilled artisan, the dose of the composition required to elicit an immune response depends on a number of factors. One of skill in the art can readily determine appropriate single dose sizes for administration based on the size of the mammal and the route of administration.

A suitable single dose of a composition comprising a vector in association with a mutant polypeptide is a dose that is capable of effectively providing a vector and/or mutant polypeptide to a given cell type, tissue, or region of the patient body in an amount effective to elicit an immune response, when administered one or more times over a suitable time period. In the case of yeast, a single dose of a yeast vehicle of the present invention is from about 0.004 YU (4x10³ cells) to about 100 YU (1x10⁹ cells), such as 0.1 YU (1x10⁶ cells) to about 100 YU (1x10⁹ cells) per dose (i.e., per organism), including any interim dose, in increments of 0.1x10⁶ cells (i.e., 1.1x10⁶, 1.2x10⁶, 1.3x10⁶, etc.). This range of doses can be effectively used in any organism of any size, including mice, monkeys, humans, etc. When the composition is administered by loading the yeast vehicle and mutant antigen into dendritic cells, a single dose of a composition described herein is from about 0.5 x10⁶ to about 40x10⁶ dendritic cells per mammal per administration. In other examples, a single dose is from about 1x10⁶ to about 20x10⁶ dendritic cells per individual, and yet other examples from about 1x10⁶ to about 10x10⁶ dendritic cells per mammal. A "boost" dose of a composition comprising a yeast vehicle as described herein may be administered when the immune response against the mutant antigen has waned, or as needed to provide an immune response or induce a memory response against a particular mutant polypeptide. Boost doses can be administered from about 1 week to several years after the original administration. In one example, an administration schedule is one in which from about 1x10⁵ to about 1x10⁹ yeast cell equivalents of a composition is administered weekly for 3 months, to weekly for 1 month (5 doses) followed by monthly administration.

In some examples, a dendritic cell composition comprising a yeast vehicle as described herein contains from about 0.5 x 10⁶ to about 40 x 10⁶ dendritic cells per single dose per patient, and in another example, from about 1 x 10⁶ to about 10 x 10⁶ dendritic cells per single dose per patient. These doses are given for a typical human or other primate. Doses suitable for other animals can be determined by those of skill in the art. For example, for a mouse, a suitable dose is from about 1 x 10⁶ to about 3x10⁶ per single dose per mouse. Other doses can be determined by the skilled artisan and is well within the ability of those of skill in the art. A composition effective to administer to a mammal contains from about 0.5 x 10⁶ to about 40 x 10⁶ dendritic cells per single dose per individual mammal.

It will be obvious to one of skill in the art that the number of doses administered to a mammal is dependent upon the nature of the yeast vehicle and the response of a mammal to the administration. Thus, it is within the scope of the present invention that a suitable number of doses includes any number required for the desired purpose. For example, repeated dosing may increase the number of T cells available to attack target cells. The dosage and frequency of the administration may be adjusted during the course of the administration as will be apparent to the skilled.

### Kits

Provided herein are kits for carrying out any of the methods described herein. Kits of the invention may comprise at least one yeast vehicle and at least one mutant polypeptide that is known to emerge or has emerged with a specific mutation in response to administration of a targeted therapeutic and/or prophylactic drug agent(s). The kit may further comprise a therapeutic and/or prophylactic drug agent. In some examples the drug agent is targeted to cancer cells. In some examples the drug agent is targeted to a virus or cells infected with a virus. The kit may further comprise instructions for carrying out a method described herein.

Kits comprising a single component will generally have the component enclosed in a container (e.g., a vial, ampoule, or other suitable storage container). Likewise, kits including more than one component may also have the reagents in containers (separately or in a mixture).

The instructions relating to the use of the kit for carrying out the invention generally describe how the contents of the kit are used to carry out the methods of the invention. Instructions supplied in the kits of the invention are typically written instructions on a label or package insert (e.g., a paper sheet included in the kit), but machine-readable instructions (e.g., instructions carried on a magnetic or optical storage disk) are also acceptable.

The invention includes a kit for immunizing a human patient against a poxvirus infection, the kit comprises an immunogenic amount of the soluble truncated poxvirus envelope protein encoded by an isolated nucleic acid encoding a vaccinia virus soluble truncated envelope protein.

Although the foregoing invention has been described in some detail by way of illustration and example for purposes of clarity of understanding, it will be apparent to those skilled in the art that certain changes and modifications may be practiced. Therefore, the descriptions and examples should not be construed as limiting the scope of the invention.

### EMBODIMENTS OF THE INVENTION

1. A composition comprising one or more of the following:
   i) a yeast vehicle comprising nucleic acid which encodes at least one mutant polypeptide, a fragment thereof that comprises a mutation, or a mimetope;
   ii) a yeast vehicle comprising at least one mutant polypeptide, a fragment thereof that comprises a mutation, or a mimetope;
   iii) a yeast vehicle in association with at least one mutant polypeptide, a fragment thereof that comprises a mutation, or a mimetope;
   iv) a yeast vehicle comprising nucleic acid which encodes at least one mutant polypeptide, a fragment thereof that comprises a mutation, or a mimetope loaded intracellularly into a dendritic cell; or
   v) a yeast vehicle and at least one mutant polypeptide, a fragment thereof that comprises a mutation, or a mimetope loaded intracellularly into a dendritic cell,
   wherein the mutant polypeptide is known to emerge or has emerged with at least one specific mutation in response to administration of a targeted therapeutic and/or prophylactic agent.
2. A composition of item 1, wherein the yeast vehicle is a whole yeast, a yeast spheroplast, a yeast cytoplast, a yeast ghost, or a subcellular yeast membrane extract or fraction thereof.
3. A composition of item 1 or 2, wherein the yeast vehicle is obtained from yeast selected from the group consisting of *Saccharomyces, Schizosaccharomyces, Kluveromyces, Hansenula, Candida* and *Pichia.*
4. A composition of any one of items 1-3, wherein the yeast vehicle is obtained from *Saccharomyces cerevisiae.*
5. A composition of any one of items 1-4, wherein the mutant polypeptide, fragment thereof that comprises a mutation, or mimetope comprises two or more epitopes.
6. A composition of any one of items 1-5, wherein the mutant polypeptide, fragment thereof that comprises a mutation, or mimetope comprises two or more epitopes comprising one or more mutations.
7. A composition of any one of items 1-6, wherein the mutant polypeptide, fragment thereof that comprises a mutation, or mimetope is encoded by a RNA or DNA virus.
8. A composition of any one of items 1-7, wherein the mutant polypeptide, fragment thereof that comprises a mutation, or mimetope is encoded by a retrovirus, flavivirus, reovirus, picornavirus, coronavirus, filovirus, rhabdovirus, bunyaviurs, orthomyxovirus, paramyxovirus, arenavirus, calicivirus, hepadnavirus, herpesvirus, poxvirus, adenovirus, parvovirus or papovavirus.
9. A composition of any one of items 1-8, wherein the mutant polypeptide, fragment thereof that comprises a mutation, or mimetope is encoded by HIV, HBV or HCV.
10. A composition of any one of items 1-9, wherein the agent is altazanavir, saquinavir, lamivudine, didnosine, embricitabine, zidovudine, stavudine, zalcitabine, abacavir, tenofovir, nevirapine, delavirdine, efavirenz, indinavir, ritonavir, nelfinavir, amprenavir, lopinavir, enfuvirtide, entecavir, adefovir, famciclovir, ganciclovir, interferon, ribavirin, thymosin-α, kinase inhibitors, nucleoside reverse transcriptase inhibitors, non-nucleoside reverse transcriptase inhibitors, viral protease inhibitors, viral entry/fusion inhibitors, viral protease inhibitors, nucleoside analogues or combinations thereof.
11. A composition of any one of items 1-10, wherein the mutant polypeptide, fragment thereof that comprises a mutation, or mimetope is encoded by HIV.
12. A composition of any one of items 1-11, wherein the mutant polypeptide, fragment thereof that comprises a mutation, or mimetope is encoded by HIV and at least one specific mutation has emerged in response to administration of an agent selected from the group consisting of nucleoside reverse-transcriptase inhibitors, non-nucleoside reverse transcriptase inhibitors, nucleoside analogues, protease inhibitors, entry/fusion inhibitors and combinations thereof.
13. A composition of any one of items 1-10, wherein the mutant polypeptide, fragment thereof that comprises a mutation, or mimetope is encoded by HCV.
14. A composition of any one of items 1-10 or 13, wherein the mutant polypeptide, fragment thereof that comprises a mutation, or mimetope is encoded by HCV and at least one specific mutation has emerged in response to administration of an agent selected from the group consisting of viral polymerase inhibitors, non-nucleoside viral polymerase inhibitors, nucleoside analogues, protease inhibitors, interferon, and combinations thereof.
15. A composition of any one of items 1-10, wherein the mutant polypeptide, fragment thereof that comprises a mutation, or mimetope is encoded by HBV.
16. A composition of any one of items 1-10 or 15, wherein the mutant polypeptide, fragment thereof that comprises a mutation, or mimetope is encoded by HBV and at least one specific mutation has emerged in response to administration of an agent selected from the group consisting of nucleoside viral polymerase inhibitors, non-nucleoside viral polymerase inhibitors, nucleoside analogues, interferon-α, thymosin-α and combinations thereof.
17. A composition of any one of items 1-6, wherein the mutant polypeptide, fragment thereof that comprises a mutation, or mimetope is encoded by an oncogene, is a tumor-associated antigen or is expressed by cancer cells.
18. A composition of any one of items 1-6 or 17, wherein the mutant polypeptide, fragment thereof that comprises a mutation, or mimetope is MAGE, MAGE3, MAGEA6, MAGEA10, NY-ESO-1, gp100, tyrosinase, EGFR, FGFR, PSA, PSMA, VEGF, PDGFR, Kit, PMSA, CEA, Her2/neu, Muc-1, hTERT, Mart1, TRP-1, TRP-2, Bcr-Abl, p53, p73, Ras, PTENSrc, p38, BRAF, adenomatous polyposis coli, myc, von Hippel landau protein, Rb-1, Rb-2, BRAC1, BRAC2, androgen receptor, Smad4, MDR1 or Flt3.
19. A composition of any one of items 1-6, 17 or 18, wherein the mutant polypeptide, fragment thereof that comprises a mutation, or mimetope is Bcr-Abl, Kit, PDGFR, EGFR, p38, Src, FGFR or Flt3.
20. A composition of any one of items 1-6 or 17-19, wherein the mutant polypeptide, fragment thereof that comprises a mutation, or mimetope comprises at least one specific mutation which has emerged in response to administration of an agent selected from the group consisting of imatinib, gefitinib, erlotinib, kinase inhibitors, SB 203580, tyrosine kinase inhibitors, or combinations thereof.
21. A composition of any one of items 1-20, comprising two mutant polypeptides, fragments thereof that comprises a mutation, or mimetopes.
22. A composition of any one of items 1-20, comprising three mutant polypeptides, fragments thereof that comprises a mutation, or mimetopes.
23. A composition of any one of items 1-22, further comprising a pharmaceutically acceptable excipient.
24. A composition of any one of items 1-23, further comprising at least one adjuvant.
25. A composition of item 24, wherein the adjuvant is an agonist of Toll-like receptor, dinucleotide CpG sequences, single- or double-stranded RNA, Freund's adjuvant, a lipid moiety, mannans, glucans, aluminum-based salts, calcium-based salts, silica, polynucleotides, toxoids, serum proteins, viral coat proteins, gamma interferon, copolymers, Ribi adjuvants, or saponins and their derivatives.
26. A composition of any one of items 1-25 for use in eliciting an immune response to a mutant polypeptide, a fragment thereof that comprises a mutation, or a mimetope in a mammal, comprising administration to the mammal of an effective amount of the composition.
27. A composition of item 26, for use in eliciting an immune response to a mutant polypeptide, a fragment thereof that comprises a mutation, or a mimetope in a mammal, comprising administration to the mammal of an effective amount of the composition in conjunction with the targeted therapeutic and/or prophylactic agent.
28. A composition of item 26 or 27, wherein the immune response is a cellular immune response.
29. A composition of item 26 or 27, wherein the immune response is a humoral immune response.
30. A composition of item 26 or 27, wherein the immune response is a cellular and humoral immune response.
31. A composition of any one of items 26-30 for use in eliciting an immune response to a mutant polypeptide, a fragment thereof that comprises a mutation, or a mimetope in a mammal, wherein the mutant polypeptide, fragment or mimetope is encoded by an oncogene, is a tumor-associated antigen or a polypeptide expressed by a cancer cell.
32. A composition of any one of items 26-30 for use in eliciting an immune response to a mutant polypeptide, a fragment thereof that comprises a mutation, or a mimetope in a mammal, wherein the mutant polypeptide, fragment or mimetope is encoded by HIV, HBV or HCV.
33. A composition of any one of items 1-25 for use in ameliorating a symptom of a disease in a mammal, comprising administration to the mammal of an effective amount of the composition, wherein the disease is associated with a mutant polypeptide that has emerged in response to administration of a targeted therapeutic and/or prophylactic agent.
34. A composition of item 33 for use in ameliorating a symptom of a disease in a mammal, wherein the disease is cancer.
35. A composition of item 33 for use in ameliorating a symptom of a disease in a mammal, wherein the disease is infection with HIV, HBV or HCV.
36. A composition of any one of items 33-35 for use in ameliorating a symptom of a disease in a mammal, comprising administration to the mammal of an effective amount of the composition in conjunction with the targeted therapeutic and/or prophylactic agent.
37. A composition of any one of items 1-25 for use in reducing resistance to an agent administered to a mammal at risk of disease or infection or subject to disease or infection, comprising administration to the mammal of an effective amount of the composition, wherein the disease or infection is associated with a mutant polypeptide that has emerged in response to administration of a targeted therapeutic and/or prophylactic agent.
38. A composition of item 37 for use in reducing resistance to an agent administered to a mammal at risk of disease or infection or subject to disease or infection, wherein the disease is cancer.
39. A composition of item 37 for use in reducing resistance to an agent administered to a mammal at risk of disease or infection or subject to disease or infection, wherein the disease is infection with HIV, HBV or HCV.
40. A composition of item 37-39 for use in reducing resistance to an agent administered to a mammal at risk of disease or infection or subject to disease or infection, comprising administration to the mammal of an effective amount of the composition in conjunction with the targeted therapeutic and/or prophylactic agent.
41. A composition of any one of items 26-40, wherein the mammal is human.
42. A method for eliciting an immune response to a mutant polypeptide, a fragment thereof that comprises a mutation, or a mimetope in a mammal comprising administration to the mammal of an effective amount of a composition of any one of items 1-25.
43. A method for eliciting an immune response to a mutant polypeptide, a fragment thereof that comprises a mutation, or a mimetope in a mammal comprising administration of an effective amount of a composition of item 42 in conjunction with a targeted therapeutic and/or prophylactic agent.
44. A composition of item 42 or 43, wherein the immune response is a cellular immune response.
45. A composition of item 42 or 43, wherein the immune response is a humoral immune response.
46. A composition of item 42 or 43, wherein the immune response is a cellular and humoral immune response.
47. A method for ameliorating a symptom of a disease in a mammal comprising administration to the mammal of an effective amount of a composition of any one of items 1-25, wherein the disease is associated with a mutant polypeptide that has emerged in response to administration of a targeted therapeutic and/or prophylactic agent.
48. A method for ameliorating a symptom of a disease in a mammal comprising administration to the mammal of an effective amount of a composition item 47 in conjunction with a targeted therapeutic and/or prophylactic agent.
49. A method for reducing resistance to an agent administered to a mammal at risk of disease or infection or subject to disease or infection, comprising administration to the mammal of an effective amount of a composition of any one of items 1-25, wherein the disease or infection is associated with a mutant polypeptide that has emerged in response to administration of a targeted therapeutic and/or prophylactic agent.
50. A method for reducing resistance to an agent administered to a mammal at risk of disease or infection or subject to disease or infection, comprising administration to the mammal of an effective amount of a composition item 49 in conjunction with a targeted therapeutic and/or prophylactic agent.
51. A method of any one of items 42-50, wherein the mammal is human.
52. The use of a composition according to any one of items 1-25 in the preparation of a medicament.
53. The use of a composition according to any one of items 1-25 in the preparation of a medicament for use in eliciting an immune response to a mutant polypeptide, a fragment thereof that comprises a mutation, or a mimetope in a mammal.
54. The use of a composition according to any one of items 1-25 in the preparation of a medicament for use in eliciting an immune response to a mutant polypeptide, a fragment thereof that comprises a mutation, or a mimetope in a mammal in conjunction with a targeted therapeutic and/or prophylactic agent.
55. The use of a composition according to any one of items 1-25 in the preparation of a medicament use in the treatment of a disease, wherein the disease is associated with a mutant polypeptide that has emerged in response to administration of a targeted therapeutic and/or prophylactic agent.
56. The use of a composition according to any one of items 1-25 in the preparation of a medicament for use in ameliorating a symptom of a disease, wherein the disease is associated with a mutant polypeptide that has emerged in response to administration of a targeted therapeutic and/or prophylactic agent.
57. The use of a composition according to any one of items 1-25 in the preparation of a medicament for use in reducing resistance to an agent administered to a mammal at risk of disease or infection or subject to disease or infection, wherein the disease or infection is associated with a mutant polypeptide that has emerged in response to administration of a targeted therapeutic and/or prophylactic agent.
58. A kit for eliciting an immune response to a mutant polypeptide, a fragment thereof that comprises a mutation, or a mimetope in a mammal, wherein the mutant polypeptide is known to emerge or has emerged with at least one specific mutation in response to administration of a targeted therapeutic and/or prophylactic agent and wherein the kit comprises a composition of any one of items 1-25.
59. A kit for eliciting an immune response to a mutant polypeptide, a fragment thereof that comprises a mutation, or a mimetope in a mammal, wherein the mutant polypeptide is known to emerge or has emerged with at least one specific mutation in response to administration ofa targeted therapeutic and/or prophylactic agent and wherein the kit comprises a yeast vehicle and at least one mutant polypeptide, fragments thereof that comprises a mutation, or mimetopes.
60. A kit of items 58 or 59, further comprising a targeted therapeutic and/or prophylactic agent.
61. A kit of items 58-60, further comprising instructional material for the use of the kit.
62. A method of preparing a yeast vehicle comprising a nucleic acid encoding for a mutant polypeptide, a fragment thereof that comprises a mutation, or a mimetope, wherein the mutant polypeptide is known to emerge or has emerged with at least one specific mutation in response to administration of a targeted therapeutic and/or prophylactic agent and wherein the nucleic acid is transfected into the yeast vehicle by a technique selected from the group consisting of diffusion, active transport, sonication, electroporation, microinjection, lipofection, adsorption and protoplast fusion.
63. A method of preparing a yeast vehicle comprising a mutant polypeptide, a fragment thereof that comprises a mutation, or a mimetope, wherein the mutant polypeptide is known to emerge or has emerged with at least one specific mutation in response to administration of a targeted therapeutic and/or prophylactic agent and wherein the mutant polypeptide, fragment thereof that comprises a mutation, or mimetope is placed into or associated with the yeast vehicle by a technique selected from the group consisting of diffusion, active transport, liposome fusion, sonication, electroporation, phagocytosis, lipofection, freeze/thaw cycling, chemical cross-linking, biological linking or mixing.
64. A method of item 62 or 63, wherein the yeast vehicle is intracellularly loaded into a dendritic cell or a macrophage.
65. A method of any one of items 62-64, wherein the yeast vehicle is intracellularly loaded into a dendritic cell.
66. A method of preparing a medicament comprising a composition of any one of items 1-25, wherein the medicament is provided for treatment of a disease related to a mutant polypeptide that has emerged in response to administration of a targeted therapeutic and/or prophylactic agent.

## Claims

1. A composition comprising one or more of the following:
(i) a yeast vehicle comprising nucleic acid which encodes at least one mutant viral polypeptide that comprises a mutation, wherein the mutation is an escape mutation that emerges as a result of administration of a targeted therapeutic and/or prophylactic agent for viral infection;
(ii) a yeast vehicle comprising at least one mutant viral polypeptide that comprises a mutation, wherein the mutation is an escape mutation that emerges as a result of administration of a targeted therapeutic and/or prophylactic agent for viral infection;
(iii) a yeast vehicle in association with at least one mutant viral polypeptide that comprises a mutation, wherein the mutation is an escape mutation that emerges as a result of administration of a targeted therapeutic and/or prophylactic agent for viral infection;
(iv) a yeast vehicle comprising nucleic acid which encodes at least one mutant viral polypeptide that comprises a mutation, wherein the mutation is an escape mutation that emerges as a result of administration of a targeted therapeutic and/or prophylactic agent for viral infection, the yeast vehicle loaded intracellularly into a dendritic cell; or
(v) a yeast vehicle and at least one mutant viral polypeptide that comprises a mutation, wherein the mutation is an escape mutation that emerges as a result of administration of a targeted therapeutic and/or prophylactic agent for viral infection, the yeast vehicle loaded intracellularly into a dendritic cell.

2. A composition of claim 1, wherein the agent is altazanavir, saquinavir, lamivudine, didnosine, embricitabine, zidovudine, stavudine, zalcitabine, abacavir, tenofovir, nevirapine, delavirdine, efavirenz, indinavir, ritonavir, nelfinavir, amprenavir, lopinavir, enfuvirtide, entecavir, adefovir, famciclovir, ganciclovir, interferon, ribavirin, thymosin-α, kinase inhibitors, nucleoside reverse transcriptase inhibitors, non-nucleoside reverse transcriptase inhibitors, viral protease inhibitors, viral entry/fusion inhibitors, viral protease inhibitors, nucleoside analogues or combinations thereof.

3. A composition of claim 1, wherein the mutant viral polypeptide is encoded by HCV.

4. A composition of claim 3, wherein the mutation emerges as a result of administration of an agent selected from the group consisting of viral polymerase inhibitors, non-nucleoside viral polymerase inhibitors, nucleoside analogues, protease inhibitors, interferon and combinations thereof.

5. A composition of claim 3, wherein the mutant viral polypeptide is from HCV NS3 protease.

6. A composition of claim 3, wherein the mutant viral polypeptide comprises one or more of the following mutations: A156S, D168V, and D168A.

7. A composition of claim 1, wherein the mutant viral polypeptide is encoded by HBV.

8. A composition of claim 7, wherein the mutation emerges as a result of administration of an agent selected from the group consisting of nucleoside viral polymerase inhibitors, non-nucleoside viral polymerase inhibitors, nucleoside analogues, interferon-α, thymosin-α and combinations thereof.

9. A composition of claim 7, wherein the mutation emerges as a result of administration of lamivudine, adefovir, or entecavir.

10. A composition of claim 7, wherein the mutant viral polypeptide is from HBV polymerase.

11. A composition of claim 7, wherein the mutant viral polypeptide comprises one or more of the following mutations: *rt*R153A, *rt*F166L (F541L), *rt*I169T, *rt*V173L (V521L), *rt*P177L, *rt*L180M (L528M), *rt*A181T (A529T), *rt*A181V, *rt*T184G, *rt*T184S, *rt*S202I, *rt*M204V/I (M552V/I), *rt*M204S (M552S), *rt*V207I, *rt*N236T, and *rt*M250V.

12. A composition of any one of claims 1-11, wherein the yeast vehicle is a whole yeast.

13. A composition of any one of claims 1-11 for use in eliciting an immune response in a mammal to the mutant viral polypeptide that comprises the escape mutation that emerges as a result of administration of a targeted therapeutic and/or prophylactic agent for viral infection.

14. A composition of any one of claims 1-11 for use in reducing resistance to a targeted therapeutic and/or prophylactic agent for viral infection administered to a mammal at risk of infection with or infected with a virus.

15. A kit for eliciting an immune response to a mutant viral polypeptide comprising an escape mutation that emerges as a result of administration of a targeted therapeutic and/or prophylactic agent for viral infection, wherein the kit comprises a composition of any one of claims 1-11.
